# EUROPÄISCHE PATENTSCHRIFT

(11) **EP 2 655 346 B1**
(45) Veröffentlichungstag und Bekanntmachung des Hinweises auf die Patenterteilung: **21.01.2015**
(21) Anmeldenummer: 11807678.5
(22) Anmeldetag: 19.12.2011
(51) Int. Cl.: C07D 251/20, C07D 403/04, A01N 43/66

(54) **VERFAHREN ZUR HERSTELLUNG VON 2-(TRIAZINYLCARBONYL)SULFONANILIDEN**
PROCESS FOR THE PREPARATION OF 2-(TRIAZINYLCARBONYL)SULFONANILIDES
PROCÉDÉ DE PRÉPARATION DE 2-(TRIAZINYLCARBONYL)SULFONANILIDES

(30) Priorität: 21.12.2010 EP 10196205; 21.12.2010 US 201061425349 P; 25.03.2011 EP 11159875; 25.03.2011 US 201161467598 P; 15.11.2011 DE 102011086382
(43) Veröffentlichungstag der Anmeldung: 30.10.2013
(73) Patentinhaber: Bayer Intellectual Property GmbH, 40789 Monheim am Rhein (DE)
(72) Erfinder: KARIG, Gunter, 65719 Hofheim am Taunus (DE); FORD, Mark, James, 61389 Schmitten (DE); SIEGEL, Konrad, 40597 Düsseldorf (DE)
(74) Vertreter: BIP Patents
(86) Internationale Anmeldenummer: PCT/EP2011/073287
(87) Internationale Veröffentlichungsnummer: WO 2012/084857

(56) Entgegenhaltungen:
- WO-A1-2005/096818
- WO-A1-2006/008159
- WO-A2-2007/031208
- JP-A- 2000 044 546
- US-A- 4 021 469
- US-A1- 2009 062 536

## Beschreibung

Die vorliegende Anmeldung betrifft ein Verfahren, dem die oxidative Ringöffnung einer Verbindung mit Oxindolstruktur zugrunde liegt, zur Herstellung von substituierten 2-(Triazinylcarbonyl)sulfonaniliden der Formel (1-1), ausgehend von N-sulfonylsubstituierten 3-Triazinyloxindolen der Formel (2-1), die so hergestellten 2-(Triazinylcarbonyl)sulfonanilide der Formel (1-1) sowie deren Verwendung als Zwischenprodukte für die Synthese von Feinchemikalien und Wirkstoffen im Bereich der Landwirtschaft

Außerdem betrifft die Erfindung ein mehrstufiges Verfahren zur Herstellung von N-Alkyl-N-[2-(1,3,5-triazin-2-ylcarbonyl)phenyl]alkansulfonamiden der Formel (4-1) ausgehend von einem 3-(Alkylsulfanyl)-1,3-dihydro-2H-indol-2-on der Formel (7-1), wobei das mehrstufige Verfahren als Teilschritt auch die genannte oxidative Ringöffnung umfasst. Als Intermediate werden bei dem mehrstufigen Verfahren, neben den 2-(Triazinylcarbonyl)sulfonaniliden der Formel (1-1), auch triazinylsubstituierte Oxindole der Formel (5-1) und N-sulfonylsubstituierte 3-Triazinyloxindole der Formel (2-1) eingesetzt.

Es ist bekannt, dass Sulfonanilide herbizide Aktivität (WO 93/09099, WO 96/41799, WO 2005/096818, WO 2007/031208 und US 2009/0062536) oder fungizide Aktivität (WO 2006/008159) aufweisen können.

Die im Stand der Technik offenbarten Sulfonanilide mit herbizider oder fungizider Wirkung umfassen als Untergruppe auch die 2-(Triazinylcarbonyl)sulfonanilide.

Die Herstellung von 2-(Triazinylcarbonyl)sulfonaniliden kann nach dem Stand der Technik auf verschiedenen Wegen erfolgen. Allerdings haben die aus dem Stand der Technik bekannten Verfahren zur Herstellung von 2-(Triazinylcarbonyl)sulfonaniliden nicht die Durchführung der Reaktion im technischen, d.h. im industriellen, Maßstab zum Ziel.

In dem Dokument US 2009/0062536 wird die Herstellung eines substituierten 2-(Triazinylcarbonyl)sulfonanilids A1, wie in Schema 1 zusammengefasst, beschrieben.

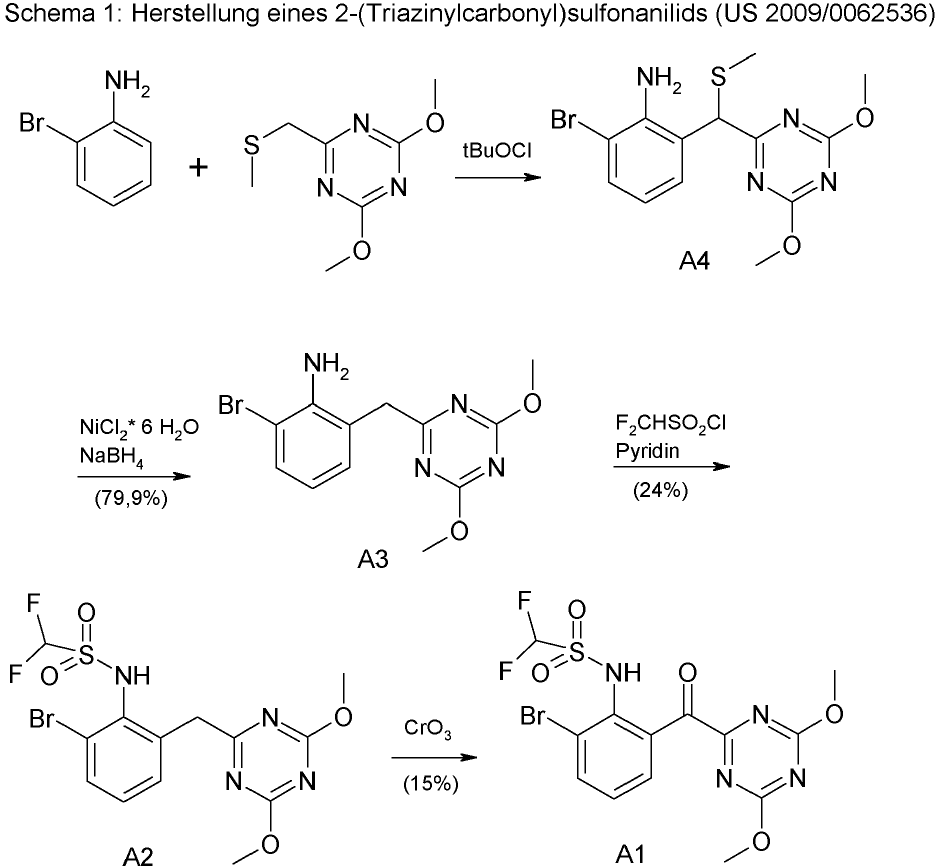

Bei dem Verfahren gemäß Schema 1 wird das substituierte 2-[(Methylsulfanyl)(1,3,5-triazin-2-yl)methyl]anilin A4 mit Nickelchlorid Hexahydrat und Natriumborhydrid zu einem 2-(1,3,5-Triazin-2-ylmethyl)anilin A3 reduziert (Synthesebeispiel 13 in US 2009/0062536). Diese Verbindung wird zu einem N-[2-(1,3,5-Triazin-2-ylmethyl)phenyl]alkansulfonamid A2 sulfonyliert (Synthesebeispiel 15 in US 2009/0062536). Anschließend wird mit 4 Äquivalenten Chrom(VI)oxid zum gewünschten Produkt A1 oxidiert (Synthesebeispiel 17 in US 2009/0062536). Die Ausbeute der in US 2009/0062536 offenbarten Oxidation beträgt 15%.

Die Verwendung des Verfahrens gemäß Schema 1 im technischen Maßstab hat den Nachteil, dass die krebserregenden Reagenzien Chrom(VI)oxid und Nickelchlorid Hexahydrat im Überschuss eingesetzt werden müssen und dennoch nur geringe Ausbeuten erzielt werden.

Neben den genannten Nachteilen in der Verfahrensführung des in Schema 1 dargestellten Herstellungswegs ist hervorzuheben, dass auch die Herstellung des in Schema 1 als Edukt eingesetzten 2-[(Methylsulfanyl)(1,3,5-triazin-2-yl)methyl]anilins A4 aufwendig ist. Die Herstellung des Eduktes A4 wird ebenfalls in US 2009/0062536 beschrieben (Synthesebeispiel 7 in US 2009/0062536). Dazu wird ein substituiertes Anilin mit tert-Butylhypochlorit und 2-[(Methylsulfanyl)methyl]-1,3,5-triazin umgesetzt.

Jedoch hat die technische Anwendung dieses Verfahrens den Nachteil, dass als Reagenz das explosive tert-Butylhypochlorit eingesetzt werden muss.

Außerdem ist das verwendete 2-[(Methylsulfanyl)methyl]-1,3,5-triazin ein Synthesebaustein, dessen Herstellung über mehrere Stufen verläuft und dessen Einsatz daher für die technische Anwendung nachteilig ist.

Der in Schema 1 dargestellte Herstellungsweg, insbesondere die Oxidation von N-[2-(1,3,5-Triazin-2-ylmethyl)phenyl]alkansulfonamiden (siehe Verbindung A2 in Schema 1), ist an ähnlichen Synthesebeispielen auch in weiteren Dokumenten beschrieben (WO 2007/031208, WO2006/008159 und WO2005/096818A1).

Das in WO 2007/031208 offenbarte Verfahren ist in Schema 2 dargestellt. Dabei handelt es sich um ein zu dem in Schema 1 gezeigten Verfahren alternatives Verfahren zur Herstellung von substituierten 2-(Triazinylcarbonyl)sulfonaniliden.

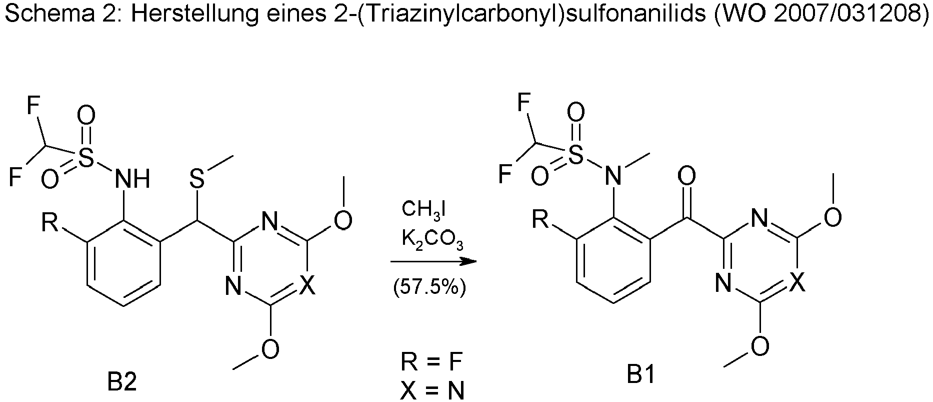

Gemäß WO 2007/031208 wird ein N-{2-[(4,6-Dimethoxy-1,3,5-triazin-2-yl)(methylsulfanyl)methyl]phenyl}methansulfonamid B2 mit Kaliumcarbonat und lodmethan 48 Stunden in einem Lösungsmittel gerührt und anschließend das gewünschte Produkt B1 isoliert (Reference Example 2 in WO 2007/031208). Jedoch beträgt die Ausbeute lediglich 57.5%.

Außerdem hat die Verwendung des in Schema 2 gezeigten Verfahrens im technischen Maßstab den Nachteil, dass die Oxidation von B2 zu B1 immer mit einer Alkylierung am Stickstoff einhergeht und daher eine nicht am Stickstoff alkylierte Verbindung auf diesem Weg nicht direkt zugänglich ist. Auch das als Alkylierungsmittel eingesetzte Methyliodid ist aus technischer Sicht wegen seines hohen Dampfdruckes und der Giftigkeit problematisch. Außerdem ist auch die lange Reaktionszeit nachteilig.

Die Ausführbarkeit der Herstellung des in Schema 2 als Edukt eingesetzten N-{2-[(4,6-Dimethoxy-1,3,5-triazin-2-yl)(methylsulfanyl)methyl]phenyl}methansulfonamids B2 wird in WO 2007/031208 nicht am Beispiel der Triazin-Verbindung (X = N), sondern lediglich am Beispiel einer Pyrimidin-Verbindung (X = CH), so wie in Schema 3 zusammengefasst, gezeigt.

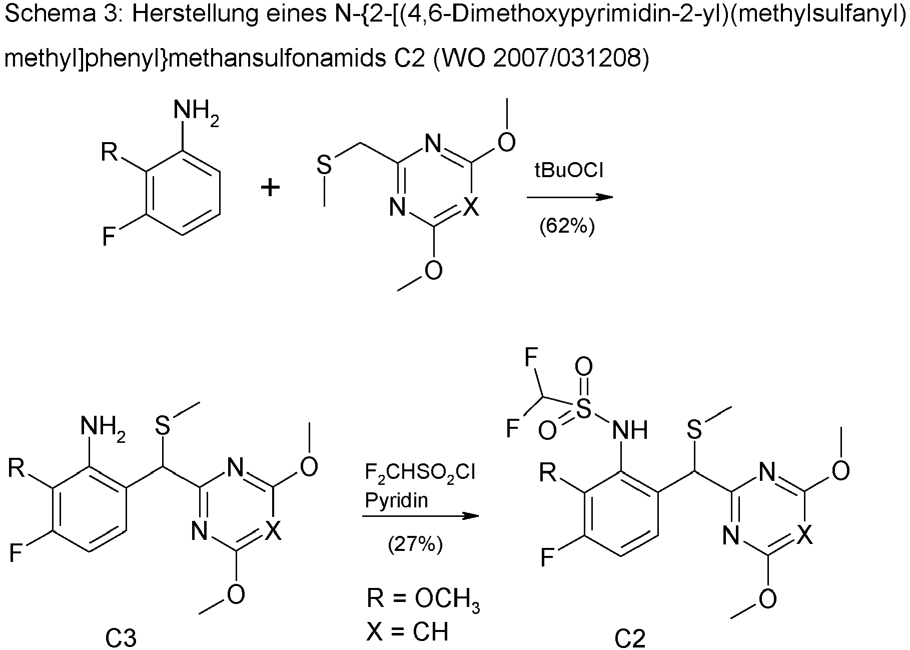

Dabei wird ein substituiertes Anilin mit tert-Butylhypochlorit und 2-[(Methylsulfanyl)methyl]-pyrimidin umgesetzt und als Zwischenprodukt ein 2-[(4,6-Dimethoxypyrimidin-2-yl)(methylsulfanyl)methyl]anilin C3 erhalten (Synthesebeispiel 10 in WO 2007/031208). Diese Verbindung wird zu einem N-{2-[(4,6-Dimethoxypyrimidin-2-yl)(methylsulfanyl)methyl]phenyl}methansulfonamid C2 sulfonyliert (Synthesebeispiel 9 in WO 2007/031208). Die Ausbeute der Sulfonylierung beträgt lediglich 27%.

In den Tabellen 5 und 6 vom Dokument WO 2007/031208 sind auch Beispiele von N-{2-[(4,6-Dimethoxy-1,3,5-triazin-2-yl)(methylsulfanyl)methyl]phenyl}methansulfon-amiden B2, bzw. 2-[(4,6-Dimethoxy-1,3,5-triazin-2-yl)(methylsulfanyl)methyl]anilinen aufgeführt, welche in ähnlicher Weise wie C2 und C3 hergestellt wurden.

Die technische Anwendung des in Schema 3 gezeigten Verfahrens hat jedoch den Nachteil, dass als Reagenz das explosive tert-Butylhypochlorit (BuOCl) eingesetzt werden muss und die Ausbeute in der Sulfonylierungsreaktion gering ist. Darüber hinaus ist das verwendete 2-[(Methylsulfanyl)methyl]-pyrimidin ein Synthesebaustein, dessen Herstellung ebenfalls über mehrere Stufen verläuft und daher für die technische Anwendung nachteilig ist.

In einem anderen vorbekannten Verfahren zur Herstellung von substituierten 2-(Triazinylcarbonyl)sulfonaniliden wird die Oxidation zum gewünschten Produkt mit Wasserstoffperoxid durchgeführt. So wird in dem bereits genannten Dokument US 2009/0062536 die Herstellung von N-{2-[(4,6-Dimethoxypyrimidin-2-yl)carbonyl]phenyl}-alkansulfonamiden D1 aus N-{2-[(4,6-Dimethoxypyrimidin-2-yl)(methylsulfanyl)methyl]-phenyl}alkansulfonamiden D2 durch Oxidation mit Wasserstoffperoxid in Eisessig (siehe Synthesebeispiele 3, 4 in US 2009/0062536), so wie in Schema 4 zusammengefasst, beschrieben.

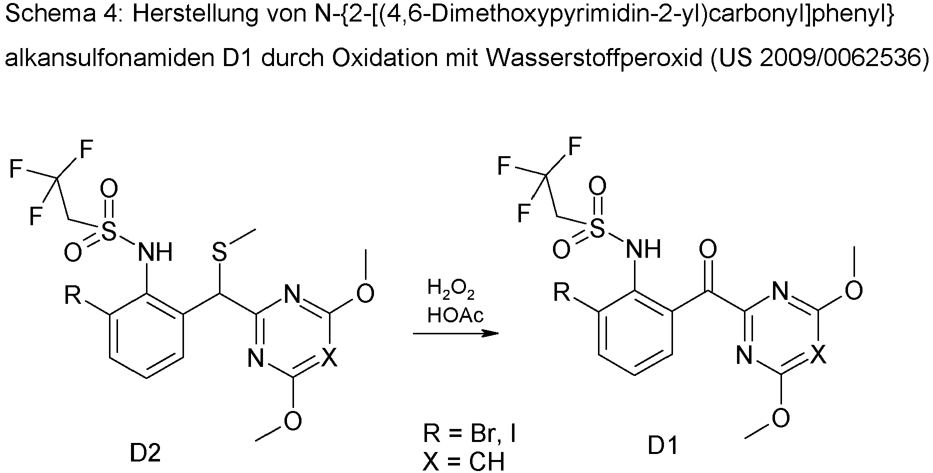

In den Tabellen 2 und 3 von Dokument US 2009/0062536 sind auch Beispiele von N-{2-[(4,6-Dimethoxy-1,3,5-triazin-2-yl)carbonyl]phenyl}-N-alkylmethansulfonamiden E1 (d.h. X = N) aufgeführt, welche in ähnlicher Weise wie D1 hergestellt wurden und immer einen Alkylrest am Stickstoff enthalten (siehe nachfolgendes Schema 5).

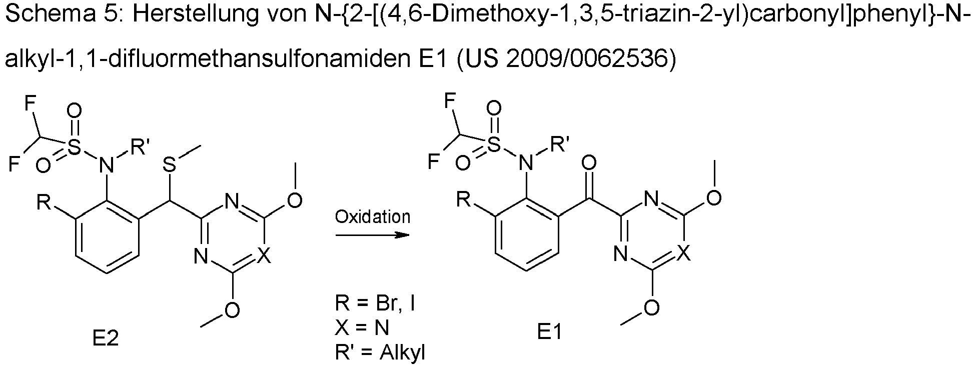

In diesem Verfahren müssen als Edukte N-{2-[(4,6-Dimethoxy-1,3,5-triazin-2-yl)(methylsulfanyl)methyl]phenyl}methansulfonamide E2 eingesetzt werden, deren Herstellung im technischen Maßstab die oben beschriebenen Nachteile hat. Die Oxidationsreaktion findet in diesem Verfahren zunächst am Schwefel statt. Unter den sauren Reaktionsbedingungen erfolgt dann die Hydrolyse zum Keton. Das Vorliegen eines oxidierbaren Schwefelsubstituenten ist daher notwendige Voraussetzung für das Verfahren.

Jedoch geht aus Dokument US 2009/0062536 nicht hervor, ob auch 2-(Triazinylcarbonyl) sulfonanilide, die nicht am Stickstoff alkyliert wurden, nach diesem Verfahren hergestellt werden können. Auch ist nicht eindeutig, ob die Oxidationsreaktion zu diesen Verbindungen mit Wasserstoffperoxid durchführbar sind, da in den ausgeführten Anwendungsbeispielen von US 2009/0062536, in denen N-{2-[(4,6-Dimethoxy-1,3,5-triazin-2-yl)carbonyl]phenyl}-N-alkylmethansulfonamide hergestellt wurden, Chromsäureanhydrid als Oxidationsmittel verwendet wurde (siehe Synthesebeispiele 17, 18 in US 2009/0062536).

Auch in WO 2007/031208 wird die Möglichkeit der Herstellung von N-{2-[(4,6-Dimethoxypyrimidin-2-yl)carbonyl]phenyl}alkansulfonamiden durch Oxidation mit Wasserstoffperoxid genannt. In den ausgeführten Anwendungsbeispielen zur Herstellung von N-{2-[(4,6-Dimethoxy-1,3,5-triazin-2-yl)carbonyl]phenyl}methan sulfonamiden wird die Oxidation allerdings ausschließlich mit Chrom(VI)oxid durchgeführt.

Somit haben die vorgenannten, aus dem Stand der Technik bekannten Verfahren den Nachteil, dass sie aus den oben genannten Gründen für eine Herstellung von 2-(Triazinylcarbonyl)sulfonaniliden im technischen, d.h. im industriellen, Maßstab aufgrund der genannten Nachteile nur bedingt geeignet sind.

Bei der Suche nach einem technisch anwendbaren Verfahren zur Herstellung von 2-(Triazinylcarbonyl)sulfonaniliden wurde im Zusammenhang mit der vorliegenden Erfindung zunächst erkannt, dass sich (N-[2-(1,3,5-Triazin-2-ylcarbonyl)phenyl]alkan-sulfonamide aus N-sulfonylsubstituierten 3-Triazinyloxindolen möglicherweise durch die in Schema 6a dargestellte oxidative Ringöffnung herstellen lassen könnten.

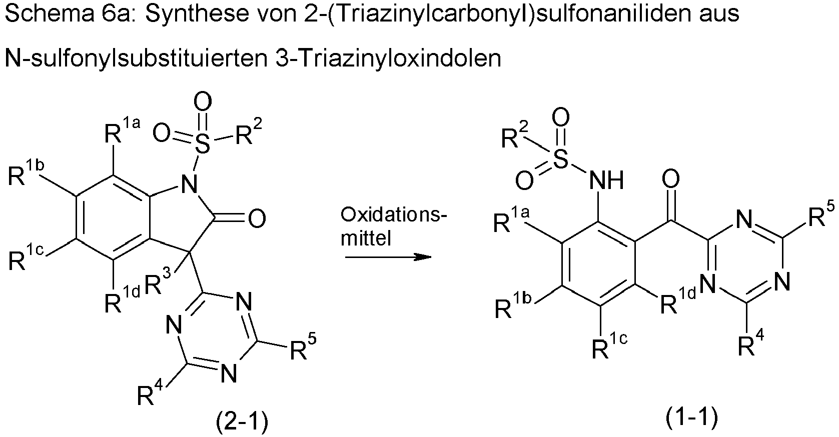

Dem allgemeinen Prinzip nach ist die oxidative Ringöffnung von 3-Phenyl-1,3-dihydro-2H-indol-2-onen zu (2-Aminophenyl)(phenyl)methanonen bekannt. Dabei ist ebenfalls bekannt, dass die Oxidation mit Sauerstoff oder mit Wasserstoffperoxid erfolgen kann.

So ist die Oxidation von 1-Methyl-3-phenyl-1,3-dihydro-2H-indol-2-on mit Sauerstoff in Gegenwart von Kobalt-Katalysatoren in der wissenschaftlichen Publikation Heterocycles, 1982, 2139 offenbart, wobei das Hauptprodukt der Oxidation das Dimere des Eduktes, nämlich 2-Methylaminobenzophenon ist. Als Produkt wird auch ein (2-Aminophenyl)(phenyl)methanon gebildet, allerdings in nur geringer Ausbeute, nämlich mit 2% bis 6%.

In Dokument US 4021469 wird die Oxidation von 3-Phenyl-1,3-dihydro-2H-indol-2-onen mit Sauerstoff in Gegenwart von Natriummethoxid in Methanol offenbart. Dabei werden zunächst die Methylcarbamate gebildet, welche in einem zweiten Reaktionsschritt nach Zugabe von Kaliumhydroxid oder Wasser, durch welches sich Natriumhydroxid bildet, durch mehrstündiges Erhitzen unter Rückfluss zum (2-Aminophenyl)(phenyl)methanon gespalten werden. Allerdings sind diese Bedingungen (siehe Beispiel 4 in US 4021469) nicht für die Umsetzung von 3-(1,3,5-Triazin-2-yl)-1,3-dihydro-2H-indol-2-onen geeignet, da der Triazinring hydrolytisch gespalten würde.

Die Oxidation von 3-Hydroxy-3-phenyl-indolin-2-onen mittels Wasserstoffperoxid wird in J. Chemical Soc., 1959, 2366 beschrieben. Die eingesetzten Edukte werden durch Addition der Phenyl-Grignard-Verbindung oder Phenyllithium an die entsprechenden Isatine hergestellt.

Jedoch ist die Herstellung der entsprechenden 3-Hydroxy-3-(1,3,5-triazin-2-yl)-1,3-dihydro-2H-indol-2-one technisch nicht sinnvoll, weil Herstellung und Umsetzung der als Edukte benötigten Triazinyl-Grignardverbindungen, bzw. der Triazinyllithium-Verbindungen, unter technischen Bedingungen schwierig sind.

Die in J. Chemical Soc., 1959, 2366 beschriebenen Bedingungen zur Oxidation, welche in Gegenwart eines großen Überschusses von wässriger NatriumhydroxidLösung bei 90°C bis 95°C durchgeführt wird, sind aufgrund der Temperatur nicht für die Umsetzung von 3-(1,3,5-Triazin-2-yl)-1,3-dihydro-2H-indol-2-onen geeignet, da der Triazinring hydrolytisch gespalten würde. Die Ausbeute beträgt 60 %.

Allerdings ist aus diesen Beispielen nicht ersichtlich, ob unter den zur Herstellung von (2-Aminophenyl)(phenyl)methanonen beschriebenen Bedingungen auch eine oxidative Ringöffnung von 1,3-Dihydro-2H-indol-2-onen, welche in 3-Position anstelle des Phenylringes einen Sechsring-Heterocyclus, insbesondere ein Triazin, aufweisen, zu den entsprechenden (2-Aminophenyl)carbonyltriazinen möglich wäre. Nicht ersichtlich ist außerdem, ob die Reaktion auch im technischen Maßstab wirtschaftlich durchführbar ist.

Die Aufgabe der Erfindung besteht vor diesem Hintergrund in der Bereitstellung eines alternativen Verfahrens zur Herstellung von 2-(Triazinylcarbonyl)sulfonaniliden in technischem Maßstab, d.h. in der Bereitstellung eines Verfahrens zur betrieblichen Herstellung von 2-(Triazinylcarbonyl)sulfonaniliden bei möglichst einfacher Verfahrensführung und möglichst hohen Ausbeuten.

Die Aufgabe wird gelöst durch ein Verfahren zur Herstellung von 2-(Triazinylcarbonyl)sulfonaniliden der Formel (1-1), 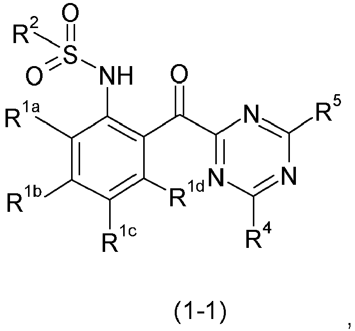 worin
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
   steht, und
R⁴ und R⁵ jeweils für Wasserstoff,
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, oder
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   stehen,
dadurch gekennzeichnet, dass
- als Edukt ein N-sulfonylsubstituiertes 3-Triazinyloxindol der Formel (2-1) 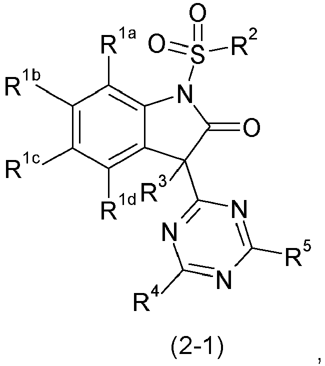 worin
   R^{1a} bis R^{1d} sowie R², R⁴ und R⁵ jeweils wie in Formel (1-1) definiert sind, und
   R³ für Wasserstoff
   steht,
   eingesetzt wird, und dass
- das in einem Lösungsmittel vorgelegte Edukt der allgemeinen Formel (2-1) in Gegenwart
   - einer Base und
   - eines Oxidationsmittels
umgesetzt wird.

Der Kerngedanke des erfindungsgemäßen Verfahrens zur Herstellung von Verbindungen der Formel (1-1) betrifft die Auswahl eines Oxindols, insbesondere eines N-sulfonylsubstituierten 3-Triazinyloxindols der Formel (2-1), als Edukt und dessen Umsetzung durch oxidative Ringöffnung.

Das in einem geeigneten Lösungsmittel vorgelegte Edukt wird zunächst mit einer geeigneten Base deprotoniert. Nach Zugabe des Oxidationsmittels findet die gewünschte oxidative Ringöffnung von Verbindungen der Formel (2-1) zu Verbindungen der Formel (1-1) statt.

Die Herstellung der als Edukte eingesetzten N-sulfonylsubstituierten 3-Triazinyloxindole der Formel (2-1) im technischen Maßstab ist in der Patentanmeldung mit der Anmeldenummer EP 111598751 beschrieben.

Besonders bevorzugt ist die Herstellung von 2-(Triazinylcarbonyl)sulfonaniliden der Formel (1-1), wobei
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
   und
R² für (C₁-C₆)-Alkyl, steht, wobei der Alkylrest ganz oder teilweise mit Fluor substituiert ist, und
R³ wie in Anspruch 1 definiert ist, und
R⁴ und R⁵ unabhängig voneinander jeweils für
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen.

Ganz besonders bevorzugt ist die Herstellung von 2-(Triazinylcarbonyl)sulfonaniliden der Formel (1-1), wobei
R^{1a} bis R^{1d} unabhängig voneinander ausgewählt sind aus der Gruppe bestehend aus Wasserstoff, Fluor, Chlor, Methoxy und
R² für Difluormethyl und R3 für Wasserstoff steht und R⁴ und R⁵ jeweils für Methoxy stehen.

Im Hinblick auf die erfindungsgemäßen Verbindungen werden die vorstehend und weiter unten verwendeten Bezeichnungen zusammenfassend erläutert. Diese sind dem Fachmann geläufig und haben insbesondere die im Folgenden erläuterten Bedeutungen:

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder lodatom.

Alkyl bedeutet einen geradkettigen oder verzweigten offenkettigen, gesättigten Kohlenwasserstoffrest.

Der Ausdruck "(C₁-C₄)Alkyl" bedeutet eine Kurzschreibweise für Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Aryl bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist.

Von der Systematik her ist Aryl in der Regel auch von dem Begriff "gegebenenfalls substituiertes Phenyl" umfasst.

Alkoxy bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Alkylthio bedeutet ein über ein Schwefelatom gebundenen Alkylrest, Alkenylthio bedeutet ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃,; Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Substituierte Reste, wie ein substituierter Alkyl-, Cycloalkyl-, Cycloalkenyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfinylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthioalkyl, Alkoxyalkyl, gegebenfalls substituiertes Mono- und Dialkylaminoalkyl und Hydroxyalkyl bedeuten.

Im Begriff "substituierte Reste", wie substituiertes Alkyl etc., sind als Substituenten neben den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und Dialkenylaminocarbonyl, Mono- und Dialkinylaminocarbonyl, Mono- und Dialkenylamino, Mono- und Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring sind auch cyclische Systeme mit jenen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Die jeweils unsubstitutierten oder substituierten Reste können verzweigt und unverzweigt sein. So umfasst zum Beispiel ein mit "C₄-Alkyl" bezeichneter Rest neben dem unverzweigten Butyl-Rest alle weiteren C₄-Isomere darunter auch tert-Butyl.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

In einer bevorzugten Ausführungsform des Verfahrens ist der Einsatz eines Schwermetallkatalysators, der als Bestandteil mindestens ein Schwermetall oder das Salz eines Schwermetalls aufweist, zur besonders effizienten Durchführung der oxidativen Ringöffnung an den als Edukt ausgewählten Verbindungen der Formel (2-1) vorgesehen. Geeignete Schwermetalle sind Vanadium, Chrom, Mangan, Eisen, Cobalt, Nickel, Kupfer, Zink, Zinn, Antimon, Wismut, Silber, Gold, Wolfram, Ruthenium und/oder Osmium.

Vorteilhafterweise bewirkt der Schwermetallkatalysator die Verringerung unerwünschter Nebenprodukte und ermöglicht so die Erhöhung der Produktausbeute der Reaktion.

So zeigte sich bei der Oxidation der im nachfolgenden Schema 6b gezeigten N-sulfonylsubstituierten 3-Triazinyloxindole der Formel (2-1) mit wässrigen Lösungen von Wasserstoffperoxid, dass, falls kein Katalysator eingesetzt wird, neben dem als Produkt gewünschten Keton der Formel (1-1) nachteiligerweise als Nebenprodukt auch ein großer Anteil des Alkohols der Formel (3-1) gebildet wird.

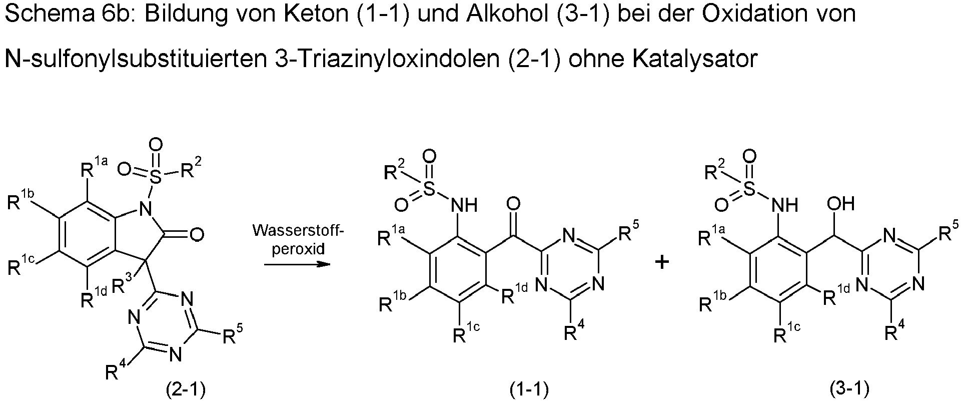

Die Bildung von Alkoholen der Formel (3-1), welche unter den Reaktionsbedingungen nicht zum Keton der Formel (1-1) weiterreagieren, führt zu Ausbeuteverlusten und ist daher nachteilig.

Aus dem zur oxidativen Ringöffnung von 3-Phenyl-1,3-dihydro-2H-indol-2-onen oben zitierten Stand der Technik ist nicht ersichtlich, dass die oxidative Ringöffnung von N-sulfonylsubstituierten 3-Triazinyloxindolen (2-1) durch Wasserstoffperoxid tatsächlich zu einer teilweisen Bildung von Alkoholen der Formel (3-1) führt (siehe Schema 6b). Auch kann dem Stand der Technik keine technische Lehre zur Vermeidung der nachteiligen Alkohol-Bildung entnommen werden.

Zum Nachweis der unerwünschten Nebenproduktbildung wurde mit dem vergleichenden Synthesebeispiel 1 Variante C (siehe BEISPIELE), die Oxidation eines N-sulfonylsubstituierten 3-Triazinyloxindols mit einer wässrigen Lösung von Wasserstoffperoxid ohne Zugabe eines Katalysators ausgeführt. Man erhält als Produkt eine Mischung, die gemäß HPLC-Analyse (Flächenprozente) zu ca. 13% aus dem Keton und zu ca. 76% aus dem Alkohol besteht. Dieses Verhältnis von Keton zu Alkohol konnte auch durch NMR bestätigt werden.

Interessanterweise wurde nun gefunden, dass sich die Bildung von Alkoholen der Formel (3-1) weitgehend oder fast völlig unterdrücken lässt, wenn die Oxidation mit Wasserstoffperoxid in Gegenwart von schwermetallhaltigen Salzen, insbesondere in Gegenwart von Eisensalzen, durchgeführt wird.

Vorteilhafterweise erlaubt das Verfahren die Herstellung von 2-(Triazinylcarbonyl)-sulfonaniliden in technischem Maßstab bei möglichst einfacher Verfahrensführung und die Erzielung hoher Ausbeuten der gewünschten Ketonverbindung durch die gezielte Vermeidung unerwünschter Nebenreaktionen, welche anstelle der gewünschten Ketone die Bildung von ungewünschten Alkoholverbindungen bewirkt.

Grundsätzlich kann eine oxidative Ringöffnung durch eine Vielzahl von Oxidationsmitteln bewirkt werden. Im Zusammenhang mit der vorliegenden Erfindung, d.h. bei der oxidativen Ringöffnung von Oxindolen, hat sich der Einsatz des Oxidationsmittels in Verbindung mit einem Katalysator als besonders vorteilhaft erwiesen.

Bevorzugtes Oxidationsmittel für die vorliegende Erfindung ist Wasserstoffperoxid in Verbindung mit einem Katalysator, der als Bestandteil mindestens ein Schwermetall oder das Salz eines Schwermetalls aufweist. Ganz besonders bevorzugt ist der Einsatz einer wässrigen Wasserstoffperoxid-Lösungen in Verbindung mit einem Katalysator, der als Bestandteil mindestens ein Schwermetall oder das Salz eines Schwermetalls aufweist.

Ein weiteres bevorzugtes Oxidationsmittel ist Kaliumpermangant. Dieses kann alleine oder in Verbindung mit einem Katalysator, der als Bestandteil mindestens ein Schwermetall oder das Salz eines Schwermetalls aufweist, eingesetzt werden.

Als Katalysatoren oder Katalysatorsysteme werden bevorzugt die nachfolgend genannten Schwermetallsalze, Schwermetallpulver, nämlich
- Eisensalze wie Eisen(II)sulfat, Eisenchlorid, sowie
- Eisenpulver, sowie
- Kupfersalze wie Kupfer(II)sulfat, Kupfer(II)chlorid, sowie
- Kupferpulver, sowie
- Mischungen aus mindestens zwei der vorgenannten Katalysatoren
eingesetzt.

Um die Stabilität des Metallkatalysators zu erhöhen, kann dem Metallpulver oder dem Metallsalz jeweils eine oder mehrere komplexbildende Substanzen, wie z.B. Pyridin-2-carbonsäure, zugesetzt werden. Bevorzugt sind dabei
- Mischungen aus Eisensalzen mit komplexbildenden Verbindungen, wie z.B. Pyridin-2-carbonsäure, sowie
- Mischungen aus Kupfersalzen mit komplexbildenden Verbindungen, wie z.B. Pyridin-2-carbonsäure, oder
- Mischungen aus mindestens zwei der vorgenannten Katalysatorsysteme.

Besonders bevorzugte Eisensalze sind Eisen(II)sulfat oder Eisenchlorid, bzw. Mischungen aus beiden Salzen. Ganz besonders bevorzugte Kupfersalze sind Kupfer(II)sulfat oder Kupfer(II)chlorid, bzw. Mischungen aus beiden Salzen.

Als komplexbildende Verbindung besonders bevorzugt ist Pyridin-2-carbonsäure.

Am meisten bevorzugt als Katalysatorsystem ist eine aus Eisen(II)sulfat und Pyridin-2-carbonsäure bestehende Mischung.

Bezüglich dieser am meisten bevorzugten Ausführungsform wird beispielhaft verwiesen auf Synthesebeispiel 1 Variante A, worin die Oxidation eines N-sulfonylsubstituierten 3-Triazinyloxindols mit wässriger Lösung von Wasserstoffperoxid unter Zugabe von Eisensulfat und Pyridin-2-carbonsäure als Katalysatorsystem ausgeführt wird. Das als Edukt eingesetzte N-sulfonylsubstituierte 3-Triazinyloxindol ist identisch mit dem in Synthesebeispiel 1 Variante C eingesetzten Edukt. Man erhält das der Formel (1-1) entsprechende Keton in einer Ausbeute von 90%. Ein Alkohol der Formel (3-1) als unerwünschtes Nebenprodukt konnte nicht nachgewiesen werden.

Als Lösungsmittel werden bevorzugt organische Lösungsmittel verwendet, die sich mit Wasser ganz oder teilweise mischen lassen. Organische, mit Wasser ganz oder teilweise mischbare Lösungsmittel, sind
- Nitrile, insbesondere Acetonitril, oder
- Alkohole, insbesondere 2-Propanol, oder
- Ketone, insbesondere Aceton.

Die genannten organischen Lösungsmittel werden bevorzugt mit Wasser gemischt eingesetzt.

Besonders bevorzugt ist die Durchführung des Verfahrens mit einer Mischung aus Acetonitril und Wasser als Lösungsmittel oder mit einer Mischung aus 2-Propanol und Wasser.

Ganz besonders bevorzugt ist die Durchführung des Verfahrens mit einer Lösungsmittel-Mischung aus Acetonitril und Wasser, in der das Verhältnis von Acetonitril und Wasser im Bereich von 2:1 bis 1:2 liegt.

Falls Wasserstoffperoxid als Oxidationsmittel verwendet wird, ist bei Verwendung von Aceton als Lösungsmittel mit der Bildung von Acetonperoxiden zu rechnen. Die Bildung von Acetonperoxid ist technisch nachteilig. Allerdings muss der genannte Nachteil nicht zwingend für alle als Lösungsmittel einsetzbaren Acetone zutreffen.

Das Verfahren wird in Gegenwart einer Base durchgeführt. Die Base bewirkt eine vollständige oder teilweise Deprotonierung der als Edukt eingesetzten N-sulfonylsubstituierten 3-Triazinyloxindole. Weil die Edukte nach der Deprotonierung in der reaktiveren Enolatform vorliegen, bewirkt der Einsatz von Basen eine insgesamt schnellere Umsetzung.

Bevorzugt werden die nachfolgend genannten Basen eingesetzt:
- Kaliumcarbonat, Natriumcarbonat oder Cäsiumcarbonat, sowie
- Kaliumhydrogencarbonat oder Natriumhydrogencarbonat, sowie
- Lithiumhydroxid, Natriumhydroxid, Kaliumhydroxid oder Bariumhydroxid , sowie
- Kaliumphosphat (K₃PO₄), Kaliumhydrogenphosphat (K₂HPO₄) oder Natriumphosphat.

Es liegt im Rahmen der Erfindung, dass als Basen auch vierfach am Stickstoff substituierte Ammoniumhydroxide der Formel N(R¹⁰)₄OH, worin die Reste R¹⁰ unabhängig voneinander ausgewählt sind aus der Gruppe bestehend aus (C₁-C₆)-Alkyl, wobei der Alkylrest verzweigt oder unverzweigt ist, und Benzyl, eingesetzt werden können.

Besonders bevorzugte Basen sind Kaliumcarbonat oder Kaliumhydrogencarbonat, die jeweils alleine oder im Gemisch eingesetzt werden.

Die Base wird entweder allein oder als Mischung aus mehreren Basen, äquimolar oder im Überschuss (1.0 bis 2.5 Äquivalente, bevorzugt 1.0 bis 1.2 Äquivalente) eingesetzt.

Auch das Oxidationsmittel wird äquimolar oder im Überschuss (1 bis 7 Äquivalente, bevorzugt 2 bis 3 Äquivalente) eingesetzt.

Der Katalysator wird äquimolar oder im Unterschuss (0.0001 bis 0.5 Äquivalente, bevorzugt 0.001 bis 0.01 Äquivalente) eingesetzt.

Die Zugabe der Reaktanden kann in einer Portion oder in mehreren Portionen über einen Zeitraum von bis zu 24 Stunden, bevorzugt bis zu 6 Stunden, insbesondere 0,05 bis 6 Stunden, erfolgen. Nach Zugabe einzelner Reaktanden kann eine Nachrührzeit (0.1 bis 12 Stunden, bevorzugt 0.5 bis 3 Stunden) vorteilhaft sein.

Die Reaktionstemperatur der Oxidation liegt im Bereich von -20°C bis 60°C, bevorzugt im Bereich von 10°C bis 40°C. Die Deprotonierung des Eduktes der Formel (2) kann bei der gleichen oder einer anderen Temperatur wie die Oxidation durchgeführt werden.

Gegebenenfalls kann die Reaktion unter Druck durchgeführt werden.

Zur Durchführung ist es vorteilhaft, wenn zuerst das N-sulfonylsubstituierte 3-Triazinyloxindol mit der Base (Gesamtmenge oder Teilmenge) in einem geeigneten Lösungsmittel vorgelegt und anschließend der Katalysator und gegebenenfalls eine weitere Menge derselben oder einer anderen Base oder einer Mischung verschiedener Basen in einer oder mehreren Portionen zugeben wird, bevor das Oxidationsmittel zugegeben wird.

Eine Zugabevariante besteht bei Verwendung von Kaliumpermanganat als Oxidationsmittel darin, dass das Oxidationsmittel und die Base in einem geeigneten Lösungsmittel vorgelegt werden und danach das N-sulfonylsubstituierte 3-Triazinyloxindol, entweder in Substanz oder in einem der genannten Lösungsmittel gelöst oder suspendiert, in einer oder mehreren Portionen zugegeben wird. Alternativ kann auch die Base in einer oder mehreren Portionen zum vorgelegten N-sulfonylsubstituierte 3-Triazinyloxindol und dem Oxidationsmittel gegeben werden. Es liegt im Rahmen der Erfindung, dass das N-sulfonylsubstituierte 3-Triazinyloxindol als Salz in die Reaktionsmischung gegeben wird. In diesem Fall kann gegebenenfalls weniger Base eingesetzt werden.

Das N-sulfonylsubstituierte 3-Triazinyloxindol und die Base können entweder in reiner Form oder miteinander vorgemischt oder in einem Lösungsmittel oder einem Lösungsmittelgemisch gelöst oder suspendiert zur Reaktionsmischung gegeben werden. Es ist möglich, dass im Verlauf der Reaktion weiteres Lösungsmittel zugegeben wird, um eine bessere Durchmischung der Reaktanden zu ermöglichen.

Abhängig von den verwendeten Reaktionsbedingungen liegt die Nachrührzeit nach Zugabe aller Reaktanden im Bereich von bis zu 96 Stunden, bevorzugt 0,05 bis 24 Stunden.

Aufarbeitung und Isolierung des gewünschten Produktes der Formel (1) kann auf verschiedene Weisen erfolgen und ist beispielsweise von der Wahl des Lösungsmittel abhängig oder ist davon abhängig, ob es sich bei dem Produkt um einen Feststoff oder um eine Flüssigkeit handelt.

Die Reaktionsmischung, welche ein festes Produkt der Formel (1) oder (1-1) enthält, wird filtriert. Das so erhaltene feste Produkt kann mit geeigneten Lösungsmitteln und /oder wässrigen Säuren gewaschen werden.

Es ist außerdem vorgesehen, dass zu der Reaktionsmischung, welche Produkt der allgemeinen Formel (1-1) enthält, ein anderes, höher siedendes Lösungsmittel gegeben wird, in welchem sich das Produkt schlechter löst, und das niedriger siedende Lösungsmittel ganz oder teilweise abdestilliert wird. Anschließend wird das als Feststoff vorliegende Produkt abfiltriert und kann mit geeigneten Lösungsmitteln und / oder wässrigen Säuren gewaschen werden.

Es liegt außerdem im Rahmen der Erfindung, dass das nach Filtration und optionalem Waschen erhaltene Produkt aus einem geeigneten Lösungsmittel oder einem Gemisch mehrerer Lösungsmittel ausgerührt wird, um das Produkt ein einer höheren Reinheit zu erhalten.

Eine andere Möglichkeit der Aufarbeitung besteht in der Extraktion der Reaktionsmischung mit einem geeigneten Lösungsmittel, aus welchem das Produkt anschließend isoliert wird.

Die Produkte der Formel (1-1) können entweder als freie Sulfonanilide (d.h. Verbindungen, die am Sulfonanilid-Stickstoff protoniert sind) oder als Salze (d.h. Verbindungen, die am Sulfonanilid-Stickstoff deprotoniert sind und ein kationisches Gegenion aufweisen), isoliert werden. Die Salze können als geeignete kationische Gegenionen Kalium, Natrium, Cäsium, Lithium, Barium oder Tetraalkylammonium enthalten.

Es ist ebenfalls vorgesehen, dass die in der Reaktionsmischung enthaltenen Produkte der Formel (1-1), bzw. der Salze, ohne vorherige Isolierung weiter zu Folgeprodukten umgesetzt werden können.
Insbesondere können die in der Reaktionsmischung enthaltenen Produkte der Formel (1-1), bzw. der Salze, mit oder ohne Isolierung zu Folgeprodukten durch Alkylierung am Sulfonanilid-Stickstoff zu N-Alkyl-N-[2-(1,3,5-triazin-2-ylcarbonyl)phenyl]alkansulfonamiden umgesetzt werden.

Die so erhältlichen N-Alkyl-N-[2-(1,3,5-triazin-2-ylcarbonyl)phenyl]alkansulfonamide der allgemeinen Formel (4-1) 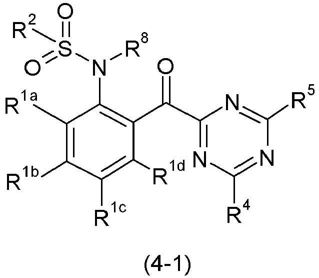 haben nachweislich herbizide Aktivität (WO 2007/031208 A2) und fungizide Aktivität (WO 2006/008159 A1).

Die Gewinnung von N-Alkyl-N-[2-(1,3,5-triazin-2-ylcarbonyl)phenyl]alkansulfonamiden der allgemeinen Formel (4-1), ausgehend von N-sulfonylsubstituierten 3-Triazinyloxindolen der Formel (2-1), ist im Vergleich zu bekannten Syntheserouten wirtschaftlich vorteilhaft.

Gegenstand der vorliegenden Erfindung ist daher auch die Verwendung von N-sulfonylsubstituierten 3-Triazinyloxindolen der Formel (2-1) 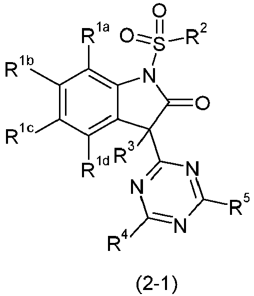 oder deren Salze (2-1 a) 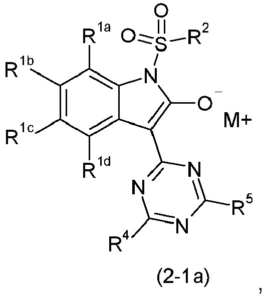 worin jeweils
R^{1a} bis R^{1d} unabhängig voneinander ausgewählt sind aus der Gruppe bestehend aus
Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
   steht, und
R³ für Wasserstoff steht, und
R⁴ und R⁵ unabhängig voneinander jeweils für
   Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   stehen,
wobei in den Salzen der allgemeinen Formel (2-1 a) M für Li, Na, K, Cs, Ba, Mg, Ca, Zn oder N(R^{c})₄, worin R^{c} = H oder C₁-C₆ Alkyl oder Benzyl ist, steht, und wobei sich die Anzahl der Gegenionen M⁺ nach der jeweiligen Ladung richtet, so dass Verbindung der allgemeinen Formel (2-1 a) insgesamt neutral sind,
als Edukte oder als Zwischenprodukte für die Synthese von Wirkstoffen im Bereich der Landwirtschaft.

Besonders bevorzugt ist die Verwendung von N-sulfonylsubstituierten 3-Triazinyloxindolen der Formel (2-1) oder Salzen der Formel (2-1 a), wobei
R^{1a} bis R^{1d} unabhängig voneinander ausgewählt sind aus der Gruppe bestehend aus Wasserstoff, Fluor, Chlor sowie aus
(C₁-C₆)-Alkyl, wobei der Alkylrest verzweigt oder unverzweigt ist,
(C₁-C₆)-Alkoxy, wobei der Alkoxyrest verzweigt oder unverzweigt ist, und
R² für Methyl, wobei das Methyl ganz oder teilweise mit Fluor substituiert ist, oder (C₃-C₇)-Cycloalkyl, wobei der Cycloalkylrest ganz oder teilweise mit Fluor substituiert ist, steht,
R³ für Wasserstoff steht, sowie
R⁴ und R⁵ unabhängig voneinander jeweils für
(C₁-C₄)-Alkyl, wobei der Alkylrest verzweigt oder unverzweigt ist,
(C₁-C₄)-Alkoxy, wobei der Alkoxyrest verzweigt oder unverzweigt ist, stehen,
wobei in den Salzen der allgemeinen Formel (2-1 a) M für Na und K steht, als Zwischenprodukte für die Synthese von Wirkstoffen im Bereich der Landwirtschaft.

Ganz besonders bevorzugt ist die Verwendung von N-sulfonylsubstituierten 3-Triazinyloxindolen der Formel (2-1) oder Salzen der Formel (2-1 a), wobei
R^{1a} bis R^{1d} unabhängig voneinander ausgewählt sind aus der Gruppe bestehend aus Wasserstoff, Fluor, Chlor, Methoxy, und
R² für Difluormethyl oder Trifluormethyl steht,
R³ für Wasserstoff steht, und
R⁴ und R⁵ unabhängig voneinander jeweils für Methoxy stehen,
als Zwischenprodukte für die Synthese von Wirkstoffen im Bereich der Landwirtschaft.

Am meisten bevorzugt ist die Verwendung von N-sulfonylsubstituierten 3-Triazinyloxindolen der Formel (2-1) oder Salze der Formel (2-1 a), wobei R^{1a} bis R^{1d} unabhängig voneinander ausgewählt sind aus der Gruppe bestehend aus Wasserstoff und Fluor, R² für Difluormethyl steht, R³ für Wasserstoff steht, und R⁴ und R⁵ unabhängig voneinander jeweils für Methoxy stehen, als Zwischenprodukte für die Synthese von Wirkstoffen im Bereich der Landwirtschaft.

Das oben beschriebene Oxidationsverfahren zur Herstellung von Verbindungen der allgemeinen Formel (1-1) wird durch den Mechanismus der oxidativen Ringöffnung charakterisiert und beruht somit auf der Auswahl von Oxindolen als Edukte, da diese einer oxidativen Ringöffnung zugänglich sind.

Die Auswahl von Verbindungen mit Oxindolstruktur als Edukte, bzw. Intermediate, kennzeichnet auch ein mehrstufiges Verfahren zur Herstellung von Verbindungen der allgemeinen Formel (1-1) und davon ausgehend von Verbindungen der allgemeinen Formel (4-1), welche sich bekannterweise durch herbizide sowie fungizide Wirkung auszeichnen.

Das oben beschriebene, auf der oxidativen Ringöffnung von Oxindolverbindungen der allgemeinen Formel (2-1) beruhende, Oxidationsverfahren zur Herstellung von Verbindungen der allgemeinen Formel (1-1) ist ein Teilschritt des in Schema 7 dargestellten mehrstufigen Verfahrens.

Die bei dem mehrstufigen Verfahren als Edukte, bzw. Intermediate, eingesetzten Oxindolverbindungen sind im nachfolgenden Schema 7 zusammengefasst und mit den allgemeinen Formeln (7-1), (6-1), (5-1), sowie (2-1) gekennzeichnet.

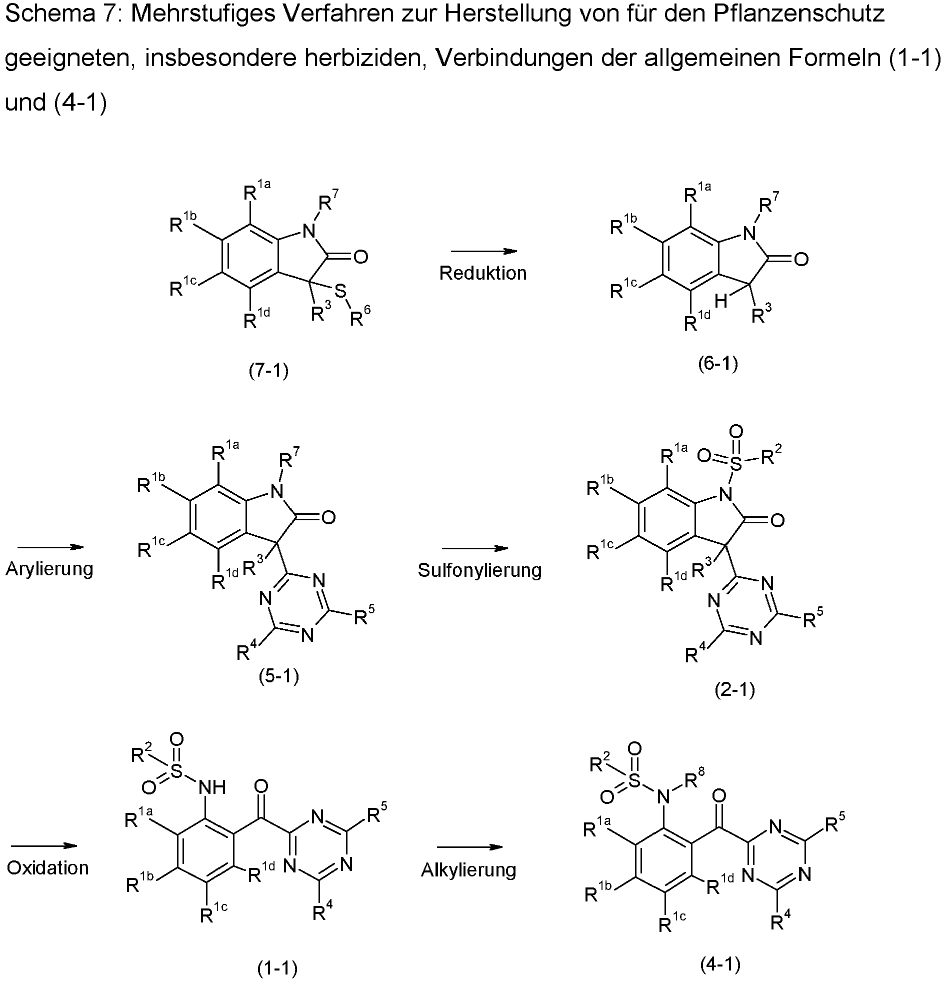

Das in Schema 7 dargestellte mehrstufige Verfahren zeichnet sich gegenüber den vorbekannten Verfahren zur Herstellung von N-Alkyl-N-[2-(1,3,5-triazin-2-ylcarbonyl)phenyl]-alkansulfonamiden (4-1) sowie 2-(Triazinylcarbonyl)sulfonaniliden (1-1) dadurch aus, dass Oxindolverbindungen als Edukte, bzw. als Intermediate, eingesetzt werden. Dies bringt den Vorteil, dass das Verfahren im Vergleich zu den vorbekannten Verfahren im technischen Maßstab durchgeführt werden kann und zugleich hohe Ausbeuten erhalten werden können.

Die Ausführung des in Schema 7 zusammengefassten Verfahrens wird nachfolgend erläutert.

Dabei wird die Reduktion, die in Schema 7 den ersten Reaktionsschritt des insgesamt fünfstufigen Verfahrens betrifft, als eigenständige Vorstufe B) behandelt.

Die übrigen der in Schema 7 zusammengefassten Reaktionsschritte, d.h. die Schritte der Arylierung, Sulfonylierung, Oxidation und Alkylierung, werden nachfolgend zusammenfassend als Verfahren A) bezeichnet.

### A) Verfahren zur Herstellung von N-Alkyl-N-[2-(1,3,5-triazin-2-ylcarbonyl)phenyl]alkansulfonamiden der Formel (4-1)

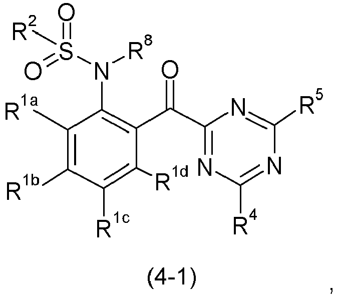

worin
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
R⁴ und R⁵ unabhängig voneinander jeweils für
   Wasserstoff,
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   stehen, und
R⁸ für
   (C₁-C₆)-Alkyl, wobei der Alkylrest unsubstituiert ist oder ganz oder teilweise mit Fluor substituiert ist,
   (C₁-C₆)-Cycloalkyl, (C₁-C₆)-Alkenyl oder (C₁-C₆)-Alkoxyalkyl, wobei jeder der genannten Reste unsubstituiert ist oder ganz oder teilweise mit Fluor substituiert ist,
   steht, wobei
ein 1,3-Dihydro-2H-indol-2-on der Formel (6-1) 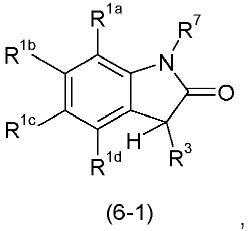 worin
R^{1a} bis R^{1d} wie für Formel (4-1) definiert sind,
R³ für Wasserstoff steht, und
R⁷ für Wasserstoff steht, in einem
ersten Schritt durch

### - Arylierung zu einem triazinylsubstituierten Oxindol der Formel (5-1)

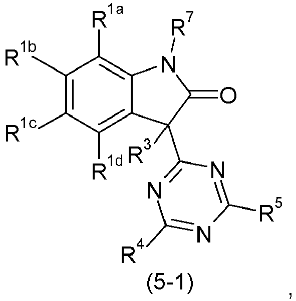

worin
R^{1a} bis R^{1d} und R⁴ und R⁵ wie für die Formel (4-1) und R³ und R⁷ wie für die Formel (6-1) definiert sind,
umgesetzt wird, und die Arylierungsprodukte der Formel (5-1) in einem
zweiten Schritt durch

### - Sulfonylierung zu N-sulfonylsubstituierten 3-Triazinyloxindolen der Formel (2-1)

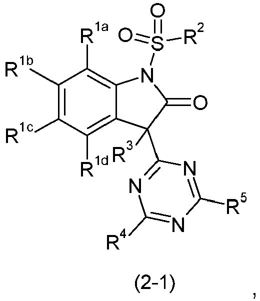

worin
R^{1a} bis R^{1d}, R² sowie R⁴ und R⁵ wie in Formel (4-1) und R³ wie für die Formel (6-1) definiert sind,
umgesetzt werden, und die Sulfonylierungsprodukte der Formel (2-1) in einem
dritten Schritt durch

### - oxidative Ringöffnung zu einem 2-(Triazinylcarbonyl)sulfonanilid der Formel (1-1)

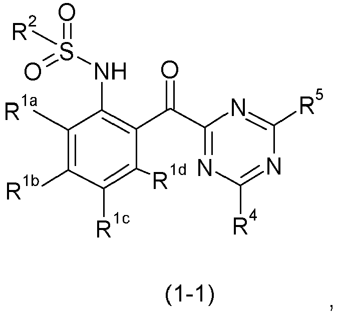

worin
R^{1a} bis R^{1d}, R² sowie R⁴ und R⁵ wie für Formel (4-1) definiert sind,
umgesetzt werden, und die Oxidationsprodukte der Formel (1-1) in einem
vierten Schritt durch

### - Alkylierung zu einem N-Alkyl-N-[2-(1,3,5-triazin-2-ylcarbonyl)phenyl] alkansulfonamid der Formel (4-1)

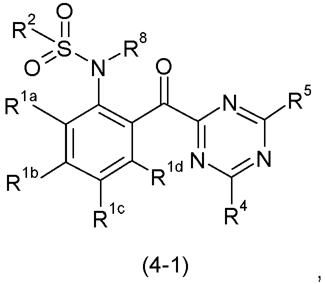

worin
R^{1a} bis R^{1d}, R², R⁴, R⁵ und R⁸ wie für Formel (4-1) definiert sind,
umgesetzt werden, wobei
als Alkylierungsreagenz
- X- R⁸, worin X für Chlor, Brom oder Iod steht und R⁸ wie oben für Formel (4-1) definiert ist, oder
- (R⁸)₂SO₄, worin R⁸ wie oben für Formel (4-1) definiert ist,
   eingesetzt wird.

Das Verfahren A) zur Herstellung von N-Alkyl-N-[2-(1,3,5-triazin-2-ylcarbonyl) phenyl]alkansulfonamiden der Formel (4-1) besteht aus vier Teilschritten, welche, mit Ausnahme der Oxidation - dem Gegenstand der vorliegenden Erfindung - Gegenstand früherer Anmeldungen sind:
- Arylierung von substituierten oder unsubstituierten 1,3-Dihydro-2H-indol-2-onen (6-1) zu triazinylsubstituierten Oxindolen (5-1). Dieses Verfahren ist im technischen Maßstab möglich und ist in der Patentanmeldung mit der Anmeldenummer EP 10196205.8 beschrieben. Im Hinblick auf die Ausführbarkeit der Arylierung wird hier auf den Inhalt der Patentanmeldung mit der Anmeldenummer EP 10196205.8 Bezug genommen.
- Sulfonylierung von triazinylsubstituierten Oxindolen (5-1) zu N-sulfonylsubstituierten 3-Triazinyloxindolen (2-1). Dieses Verfahren ist im technischen Maßstab möglich und ist in der Patentanmeldung mit der Anmeldenummer EP 11159875.1 beschrieben. Im Hinblick auf die Ausführbarkeit der Sulfonylierung wird hier auf den Inhalt der Patentanmeldung mit der Anmeldenummer EP 11159875.1 Bezug genommen.
- Oxidative Ringöffnung von N-sulfonylsubstituierten 3-Triazinyloxindolen (2-1) zu 2-(Triazinylcarbonyl)sulfonaniliden (1-1). Dieses Verfahren ist im technischen Maßstab möglich und ist Gegenstand der vorliegenden Erfindung.
- Alkylierung von 2-(Triazinylcarbonyl)sulfonaniliden (1-1) zu N-Alkyl-N-[2-(1,3,5-triazin-2-ylcarbonyl)phenyl]alkansulfonamiden (4-1). Dieses Verfahren ist in der Patentanmeldung mit der Anmeldenummer WO 2006/008159 A1 beschrieben. Im Hinblick auf die Ausführbarkeit der Alkylierung wird hier auf den Inhalt der Patentanmeldung mit der Anmeldenummer WO 2006/008159 A1 Bezug genommen.

Die Arylierung ist dadurch gekennzeichnet, dass sie in Gegenwart
- eines Carbonats, oder
- eines Hydroxids, oder
- eines Phosphats, oder
- in einer mindestens zwei der vorgenannten Basen umfassenden Mischung durchgeführt wird.

Bevorzugt werden bei der Arylierung als Basen Kaliumcarbonat, Natriumcarbonat, Natriumhydroxid, Kaliumhydroxid oder eine mindestens zwei-komponentige Mischung bestehend aus mindestens einem der beiden Carbonate: Kaliumcarbonat und Natriumcarbonat sowie aus mindestens einem der beiden Hydroxide: Kaliumhydroxid oder Natriumhydroxid, eingesetzt.

Die Sulfonylierung ist dadurch gekennzeichnet, dass sie in Gegenwart
- einer in 1-Position substituierten Imidazol-Base, oder
- eines Basen-Gemischs, das mindestens eine in 1-Position substituierte Imidazol-Base enthält,
erfolgt.

Besonders bevorzugte Imidazol-Basen für die Durchführung der Sulfonylierung sind 1-Methyl-1 H-Imidazol, 1-Butyl-1 H-Imidazol oder 1-Benzyl-1 H-Imidazol, die einzeln oder im Gemisch eingesetzt werden können, wobei die Verwendung von 1-Methyl-1 H-Imidazol ganz besonders bevorzugt ist.

Die Alkylierung kann mit gängigen Alkylierungsmitteln erfolgen. Im Falle einer Methylierung wird bevorzugt Dimethylsulfat eingesetzt.

Die herbizide Wirkung (siehe WO 2007/031208 A2) und fungizide Wirkung (siehe WO 2006/008159 A1) von N-Alkyl-N-[2-(1,3,5-triazin-2-ylcarbonyl)phenyl]alkansulfonamiden der Formel (4-1) ist seit längerem bekannt.

Somit wird durch die vorgenannten Angaben zur Ausführbarkeit der im Verfahren A) zusammengefassten Reaktionen, bestehend aus Arylierung, Sulfonylierung, Oxidation und Alkylierung, die Eignung von Oxindolen der Formeln (6-1), (5-1), (2-1) und Verbindungen der Formel (1-1) zur Herstellung von Pflanzenschutzmitteln der Formel (4-1) belegt.

### B) Verfahren zur Herstellung von N-Alkyl-N-[2-(1,3,5-triazin-2-ylcarbonyl)phenyl] alkansulfonamiden der Formel (4-1), wobei die als Edukt eingesetzten Verbindungen der Formel (6-1) in einem vorhergehenden Verfahrensschritt hergestellt werden, bei welchem ausgehend von einem 3-(Alkylsulfanyl)-1,3-dihydro-2H-indol-2-on der Formel (7-1),

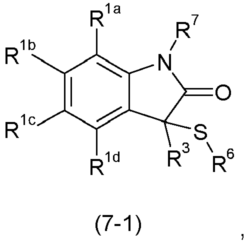

worin
R^{1a} bis R^{1d} wie für Formel (4-1) definiert sind,
R³ für Wasserstoff steht,
R⁷ für Wasserstoff steht, und
R⁶ ein unsubstituiertes oder substituiertes (C₁-C₁₄)-Alkyl, (C₃-C₇)-Cycloalkyl, Benzyl
   oder ein CH₂-C(O)O-(C₁-C₆)-Alkyl ist,
durch

### - Reduktion zu einem 1,3-Dihydro-2H-indol-2-on (6-1)

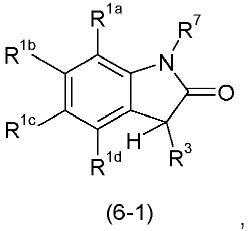

worin
R^{1a} bis R^{1d}, R³ und R⁷ wie für Formel (7-1) definiert sind,
umgewandelt wird.

Das Verfahren B) betrifft die Reduktion von substituierten oder unsubstituierten 3-(Alkylsulfanyl)-1,3-dihydro-2H-indol-2-onen (7-1) zu substituierten oder unsubstituierten 1,3-Dihydro-2H-indol-2-onen (6-1). Dieses Verfahren ist im technischen Maßstab möglich und ist in der Patentanmeldung mit der Anmeldenummer EP 10162381.7 beschrieben. Im Hinblick auf die Ausführbarkeit der Reduktion wird hier auf den Inhalt der Patentanmeldung mit der Anmeldenummer EP 10162381.7 Bezug genommen.

Die Reduktion ist dadurch gekennzeichnet, dass
a) eine Verbindung der Formel (7-1) in einem polaren Lösungsmittel gelöst oder suspendiert wird,
b) der Lösung oder der Suspension ein schwefelhaltiges Salz zugesetzt wird, und
c) das Reaktionsgemisch bei einer Temperatur, die maximal der Siedetemparatur des polaren Lösungsmittels entspricht, unter Rückfluss erwärmt wird.

Als schwefelhaltige Salze besonders bevorzugt sind Natriumsalze, ausgewählt aus der Gruppe bestehend aus Natriumbisulfit, Natriumsulfit, Natriumthionit, Natriumdithionit und Natriumthiosulfat.

Beschrieben sind auch die durch das oben erläuterte Oxidationsverfahren, d.h. die oxidative Ringöffnung von Oxindolverbindungen der Formel (2-1), hergestellten 2-(Triazinylcarbonyl)sulfonanilide der Formel (1-1) 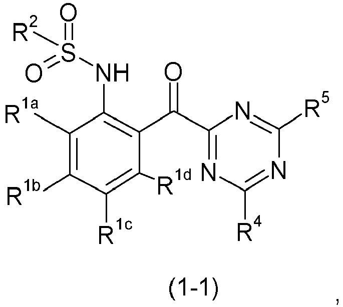 worin
R^{1a} bis R^{1d}, R², R⁴ und R⁵ wie für Formel (4-1) definiert sind, d.h.
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
R⁴ und R⁵ unabhängig voneinander jeweils für
   Wasserstoff,
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen.

Die vorgenannten Verbindungen der Formel (1-1) sind wichtige Intermediate in dem oben erläuterten Verfahren zur Herstellung von Herbiziden, bzw. Fungiziden der Formel (4-1). Die durch oxidative Ringöffnung von Oxindolverbindungen der Formel (2-1) und anschließende Alkylierung hergestellten 2-(Triazinylcarbonyl)sulfonanilide der Formel (1-1) sind ebenfalls beschrieben.

### BEISPIELE

Die nachfolgenden Beispiele erläutern die Erfindung näher, jedoch ohne deren Gegenstand auf diese Beispiele einzuschränken.

In den nachfolgenden Beispielen beziehen sich Mengenangaben auf das Gewicht, sofern nichts anderes speziell definiert ist. In der Beschreibung wurde hierfür analog die Abkürzung Gew.% = Gewichtsprozent verwendet. Für Maßeinheiten, physikalische Größen und ähnliches werden übliche Abkürzungen verwendet, beispielsweise h = Stunde(n), mpt. = Schmelzpunkt (Smp.), l = Liter, ml = Milliliter, g = Gramm, min = Minute(n), in vacuo = "im Vakuum" = unter reduziertem Druck, d. Th. = Prozent Ausbeute nach der Theorie, RT = Raumtemperatur, eq. = Äquivalente.

Die Kupplungsmuster in den NMR-Spektren werden so beschrieben, wie sie erscheinen.

Anteile auf der Grundlage der HPLC-Analytik werden, wenn nichts anderes angegeben ist, in relativen Flächenprozenten wiedergegeben.

Prozentangaben in der LC-MS-Analytik beziehen sich auf den relativen Anteil der jeweiligen Komponente im Chromatogramm.

### Beispiel 1:

### Herstellung von N-{2-[(4,6-Dimethoxy-1,3,5-triazin-2-yl)carbonyl]-6-fluorphenyl}-1,1-difluormethansulfonamid

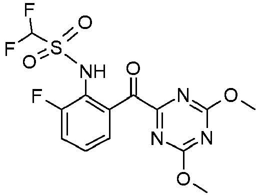

Variante A: (Oxidation mit Wasserstoffperoxid und Eisensulfat in -Wasser/Acetonitril): 1-[(Difluormethyl)sulfonyl]-3-(4,6-dimethoxy-1,3,5-triazin-2-yl)-7-fluor-1H-indol-2-ol (100g) wird in 330 ml Acetonitril vorgelegt und die Suspension auf 35°C erwärmt. Eine Lösung von Kaliumhydrogencarbonat (25,4g) in 145 ml Wasser wird innerhalb 45 min zugetropft. Eisen(II)sulfat Heptahydrat (395 mg) und Pyridin-2-carbonsäure (175 mg) werden in 1 ml Wasser vorgemischt und zum Ansatz gegeben. Innerhalb von 135 min wird Wasserstoffperoxid (35% in Wasser, 58g) zugetropft und die Innentemperatur bei 25°C-28°C gehalten. Es wird 170 min nachgerührt, Natriumsulfit (3g) und Kaliumhydrogencarbonat (3g) zugegeben, der Ansatz bei 40°C im Vakuum auf 282g eingeengt und vom festen Rückstand abfiltriert. Zur Lösung wird 2-Propanol (150 ml) gegeben und mit Salzsäure auf pH 2 eingestellt, so dass ein Feststoff ausfällt. Nach Zugabe von 160 ml Wasser wird abfiltriert, mit 100 ml 2-Propanol / Wasser (1:4) und 200 ml Wasser gewaschen und der Feststoff getrocknet. Man erhält N-{2-[(4,6-Dimethoxy-1,3,5-triazin-2-yl)carbonyl]-6-fluorphenyl}-1,1-difluormethansulfonamid in einer Reinheit von 97% (86,2g, 90% d. Th).

LC-MS: M+H = 393 (96%). 1 H-NMR (400 MHz, CDCl₃): δ (ppm) = 9.2 (breites s, 1 H), 7.54 (d, 1 H), 7.47 (t, 1 H), 7.34 (dt, 1 H), 6.48 (t, 1 H), 4.12 (s, 6H).

### Variante B (Oxidation mit Kaliumpermanganat):

Kaliumpermanganat (184 mg) und Kaliumcarbonat (96 mg) werden in 2 ml einer Mischung aus Wasser und Acetonitril (1:1) bei 0°C vorgelegt. Unter Kühlung wird 1-[(Difluormethyl)sulfonyl]-3-(4,6-dimethoxy-1,3,5-triazin-2-yl)-7-fluor-1H-indol-2-ol (186 mg) als Feststoff portionsweise innerhalb 20 min zugegeben und der Ansatz 90 min nachgerührt. Zur Aufarbeitung wird verdünnte Schwefelsäure (20% in Wasser, 2 ml) zugegeben, kurz bis zum Ende der Gasentwicklung gerührt und der Ansatz in eine Lösung von Natriumsulfit (10% in Wasser, 3 ml) getropft. Das organische Lösungsmittel wird im Vakuum weitgehend entfernt und der wässrige Rückstand mit Dichlormethan extrahiert. Die organische Phase wird einmal mit einer Lösung von Ammoniumchlorid in Wasser gewaschen und eingeengt. Man erhält N-{2-[(4,6-Dimethoxy-1,3,5-triazin-2-yl)carbonyl]-6-fluorphenyl}-1,1-difluormethansulfonamid in einer HPLC-Reinheit von >99% (123 mg, 72% d. Th). Das NMR stimmt mit dem des in Variante A erhaltenen Produktes überein.

### Variante C (=Vergleichsbeispiel 1C -Oxidation mit Wasserstoffperoxid ohne Katalysator):

1-[(Difluormethyl)sulfonyl]-3-(4,6-dimethoxy-1,3,5-triazin-2-yl)-7-fluor-1H-indol-2-ol (60g) wird in 165 ml Acetonitril vorgelegt und die Suspension auf 35°C erwärmt. Eine Lösung von Kaliumhydrogencarbonat (14,8g) in 74 ml Wasser wird zugetropft und der Ansatz innerhalb 30 min auf 35°C erwärmt und 10 min bei 35°C nachgerührt. Man kühlt wieder auf 25°C und tropft Wasserstoffperoxid (35% in Wasser, 34g) innerhalb 4 Stunden zu, wobei die Innentemperatur bei 25°C-27°C gehalten wird. Es entsteht eine klare, homogene Lösung. Der Ansatz steht über Nacht bei 22°C. Nach HPLC (210 nm, Angaben in Flächenprozent) ist 15% der Titelverbindung und 78% Alkohol (N-{2-[(4,6-Dimethoxy-1,3,5-triazin-2-yl)(hydroxy)methyl]-6-fluorphenyl}-1,1-difluormethansulfonamid) entstanden. Man gibt portionsweise Natriumsulfit (18g) und anschließend Kaliumhydrogencarbonat (19g) zum Ansatz und engt bei 40°C im Vakuum ein, wobei 168 ml Destillat entnommen werden. Da eine Isolierung des Produktgemisches als filtrierbaren Feststoff mit 2-Propanol wie in Beispiel 1 Variante A nicht möglich ist, wird der Ansatz mit Wasser auf ein Volumen von 1 L aufgefüllt, auf 5°C gekühlt, mit Salzsäure auf pH 2 gestellt, der ausgefallene Feststoff abfiltriert und mit 1 L Wasser gewaschen. Man erhält 50,1g einer Mischung, welche nach HPLC aus der Titelverbindung (14% Fläche) und dem Alkohol N-{2-[(4,6-Dimethoxy-1,3,5-triazin-2-yl)(hydroxy)methyl]-6-fluorphenyl}-1,1-difluormethansulfonamid (82% Fläche) besteht. Die Ausbeute beträgt 13% d. Th. (Titelverbindung) und 76% d. Th. (Alkohol). Das NMR der Mischung bestätigt das Vorhandensein von Titelverbindung (ca. 12%) und Alkohol (ca. 88%).

1H-NMR (400 MHz, CDCl₃) von N-{2-[(4,6-Dimethoxy-1,3,5-triazin-2-yl)(hydroxy)methyl]-6-fluorphenyl}-1,1-difluormethansulfonamid: δ (ppm) = 9.4 (breites s, 1H), 7.48 (d, 1H), 7.31 (dt, 1H), 7.14 (t, 1 H), 6.57 (t, 1 H), 6.10 (s, 1 H), 4.7 (breites s, 1H), 4.09 (s, 6H).

Die Synthesebeispiele 2 bis 9 wurden durch Oxidation mit Wasserstoffperoxid in Acetonitril / Wasser in Gegenwart von Eisen(II)sulfat Heptahydrat und Pyridin-2-carbonsäure ausgeführt.

### Vergleichsbeispiel 2: N-{2-[(4,6-Dimethoxy-1,3,5-triazin-2-yl)carbonyl]-4-methoxyphenyl}-1,1-difluormethansulfonamid

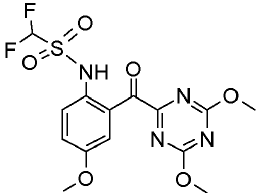

LC-MS: M+H = 405 (100%). 1 H-NMR (400 MHz, CDCl₃): δ (ppm) = 10.5 (breites s, 1 H), 7.78 (d, 1 H), 7.19 (dd. 1 H), 7.14 (d, 1 H), 6.29 (t, 1 H), 4.12 (s, 6H), 3.78 (s, 3H).

### Vergleichsbeispiel 3: N-{2-[(4,6-Dimethoxy-1,3,5-triazin-2-yl)carbonyl]-4,6-difluorphenyl}-1,1-difluormethansulfonamid

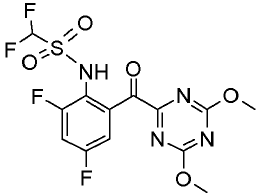

LC-MS: M+H = 411 (91%). 1 H-NMR (400 MHz, CDCl₃): δ (ppm) = 8.2 (breites s, 1 H), 7.35 (dd, 1 H), 7.21 (dt, 1 H), 6.35 (t, 1H), 4.12 (s, 6H).

### Vergleichsbeispiel 4: N-{2-[(4,6-Dimethoxy-1,3,5-triazin-2-yl)carbonyl]-6-methoxyphenyl}-1,1-difluormethansulfonamid

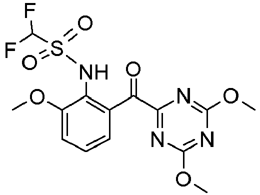

LC-MS: M+H = 405 (98%). 1 H-NMR (400 MHz, CDCl₃): δ (ppm) = 8.6 (breites s, 1 H), 7.30-7.35 (m, 2H), 7.24 (dd, 1H), 6.52 (t, 1H), 4.11 (s, 6H), 3.96 (s, 3H).

### Vergleichsbeispiel 5: N-{2-[(4,6-Diethoxy-1,3,5-triazin-2-yl)carbonyl]-6-fluorphenyl}-1,1-difluormethansulfonamid

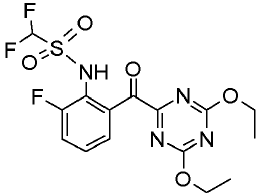

LC-MS: M-H = 419 (100%). 1 H-NMR (600 MHz, CDCl₃): δ (ppm) = 9.2 (breites s, 1 H), 7.56 (d, 1 H), 7.46 (t, 1 H), 7.35 (dt, 1 H), 6.50 (t, 1 H), 4.55 (q, 4H), 1.46 (t, 6H).

### Vergleichsbeispiel 6: 1,1-Difluor-N-{2-fluor-6-[(4-methoxy-6-methyl-1,3,5-triazin-2-yl)carbonyl]phenyl}methansulfonamid

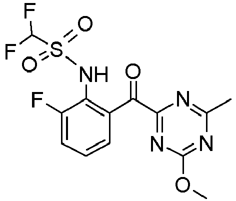

LC-MS: M+H = 377 (100%). 1 H-NMR (600 MHz, CDCl₃): δ (ppm) = 9.2 (breites s, 1 H), 7.52 (d, 1 H), 7.48 (t, 1 H), 7.36 (dt, 1 H), 6.50 (t, 1 H), 4.11 (s, 3H), 2.72 (s, 3H).

### Vergleichsbeispiel 7: N-{2-[(4,6-Dimethoxy-1,3,5-triazin-2-yl)carbonyl]-6-fluorphenyl}-1,1,1-trifluormethansulfonamid

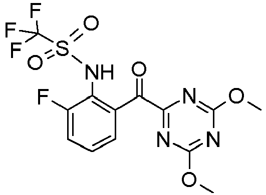

LC-MS: M+H = 411 (88%). 1 H-NMR (400 MHz, CDCl₃): δ (ppm) = 8.7 (breites s, 1 H), 7.55 (dt, 1 H), 7.40-7.50 (m, 2H), 4.12 (s, 6H).

### Vergleichsbeispiel 8: N-{2-Chlor-6-[(4,6-dimethoxy-1,3,5-triazin-2-yl)carbonyl]phenyl}-1,1-difluormethansulfonamid

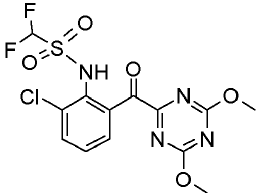

LC-MS: M+H = 409, 411 (100%). 1 H-NMR (400 MHz, DMSO-D₆): δ (ppm) = 11.0 (breites s, 1 H), 7.88 (dd, 1 H), 7.75 (dd, 1 H), 7.58 (t, 1 H), 6.93 (t, 1 H), 3.96 (s, 6H).

Die Synthesebeispiele 9 und 10 wurden durch Oxidation mit Kaliumpermanganat in Acetonitril / Wasser ausgeführt.

### Vergleichsbeispiel 9:

### N-{2-[(4,6-Dimethoxy-1,3,5-triazin-2-yl)carbonyl]-4-fluorphenyl}-1,1-difluormethansulfonamid

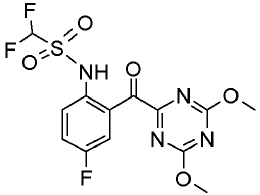

LC-MS: M+H = 393 (96%). 1 H-NMR (400 MHz, CDCl₃): δ (ppm) = 10.8 (breites s, 1 H), 7.87 (dd, 1 H), 7.34-7-42 (m, 2H), 6.33 (t, 1 H), 4.13 (s, 6H).

### Vergleichsbeispiel 10: N-{2-[(4,6-Dimethoxy-1,3,5-triazin-2-yl)carbonyl]phenyl}-1,1-difluormethansulfonamid

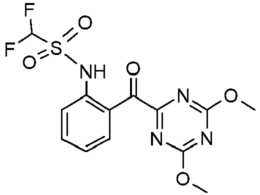

LC-MS: M+H = 375 (90%). 1 H-NMR (400 MHz, CDCl₃): δ (ppm) = 11.1 (breites s, 1 H), 7.88 (d, 1 H), 7.61-7.68 (m, 2H), 7.18 (t, 1 H), 6.34 (t, 1 H), 4.12 (s, 6H).

## Patentansprüche

1. Verfahren zur Herstellung von 2-(Triazinylcarbonyl)sulfonaniliden der Formel (1-1), 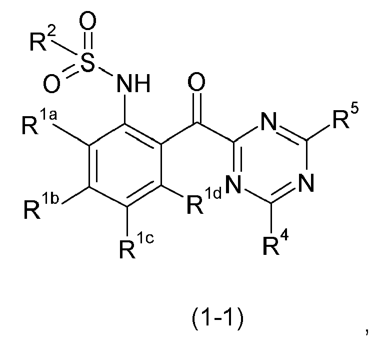 worin
R^{1a} bis R^{1d} unabhängig voneinander ausgewählt sind aus der Gruppe bestehend aus
Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
(C₁-C₆)-Alkyl, wobei der Alkylrest unsubstituiert ist oder ganz oder teilweise mit Fluor substituiert ist, oder
(C₃-C₇)-Cycloalkyl, wobei der Cycloalkylrest unsubstituiert ist oder ganz oder teilweise mit Fluor substituiert ist,
steht, und
R⁴ und R⁵ jeweils für Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, oder
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
**dadurch gekennzeichnet, dass**
- als Edukt ein N-sulfonylsubstituiertes 3-Triazinyloxindol der Formel (2-1) 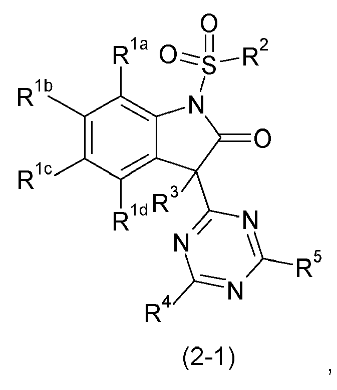 worin
R^{1a} bis R^{1d} sowie R², R⁴ und R⁵ jeweils wie in Formel (1-1) definiert sind, und
R³ für Wasserstoff
steht,
eingesetzt wird, und dass
- das in einem Lösungsmittel vorgelegte Edukt der allgemeinen Formel (2-1) in Gegenwart
- einer Base und
- eines Oxidationsmittels
umgesetzt wird.

2. Verfahren zur Herstellung von 2-(Triazinylcarbonyl)sulfonaniliden der Formel (1-1) nach Anspruch 1, wobei
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, und
R² für (C₁-C₆)-Alkyl, steht, wobei der Alkylrest ganz oder teilweise mit Fluor substituiert ist, und
R³ wie in Anspruch 1 definiert ist, und
R⁴ und R⁵ unabhängig voneinander jeweils für
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen.

3. Verfahren zur Herstellung von 2-(Triazinylcarbonyl)sulfonaniliden der Formel (1-1) nach einem der Ansprüche 1 oder 2, wobei
R^{1a} bis R^{1d} unabhängig voneinander ausgewählt sind aus der Gruppe bestehend aus Wasserstoff, Fluor, Chlor, Methoxy, und
R² für Difluormethyl und R3 für Wasserstoff steht, und
R⁴ und R⁵ jeweils für Methoxy stehen.

4. Verfahren zur Herstellung von 2-(Triazinylcarbonyl)sulfonaniliden der Formel (1-1) nach einem der Ansprüche 1 bis 3, **dadurch gekennzeichnet, dass** das Oxidationsmittel in Kombination mit einem Katalysator, der als Bestandteil mindestens ein Schwermetall oder das Salz eines Schwermetalls aufweist, eingesetzt wird.

5. Verfahren zur Herstellung von 2-(Triazinylcarbonyl)sulfonaniliden der Formel (1-1) nach Anspruch 4, **dadurch gekennzeichnet, dass** als Oxidationsmittel Wasserstoffperoxid in Verbindung mit einem Katalysator, der als Bestandteil mindestens ein Schwermetall oder das Salz eines Schwermetalls aufweist, eingesetzt wird.

6. Verfahren zur Herstellung von 2-(Triazinylcarbonyl)sulfonaniliden der Formel (1-1) nach Anspruch 4, **dadurch gekennzeichnet, dass** als Oxidationsmittel Kaliumpermanganat ohne oder mit einem Katalysator, der als Bestandteil mindestens ein Schwermetall oder das Salz eines Schwermetalls aufweist, eingesetzt wird.

7. Verfahren zur Herstellung von 2-(Triazinylcarbonyl)sulfonaniliden der Formel (1-1) nach einem der Ansprüche 1 bis 6, **dadurch gekennzeichnet, dass** als Katalysator
- Eisensalze, Eisenpulver, Kupfersalze oder Kupferpulver, oder
- Mischungen aus mindestens zwei der zuvor genannten Katalysatoren eingesetzt werden.

8. Verfahren zur Herstellung von 2-(Triazinylcarbonyl)sulfonaniliden der Formel (1-1) nach einem der Ansprüche 4 bis 7, **dadurch gekennzeichnet, dass** als Katalysator
- ein oder mehrere Eisensalze zusammen mit komplexbildenden Verbindungen, oder
- ein oder mehrer Kupfersalze zusammen mit komplexbildenden Verbindungen, oder
- Mischungen aus mindestens zwei der vorgenannten Katalysatorsysteme eingesetzt wird.

9. Verfahren zur Herstellung von 2-(Triazinylcarbonyl)sulfonaniliden der Formel (1-1) nach einem der Ansprüche 7 und 8, **dadurch gekennzeichnet, dass**, als
- Eisensalze Eisen(II)sulfat und/oder Eisenchlorid, und als
- Kupfersalze Kupfer(II)sulfat und/oder Kupfer(II)chlorid
eingesetzt werden.

10. Verfahren zur Herstellung von 2-(Triazinylcarbonyl)sulfonaniliden der Formel (1-1) nach einem der Ansprüche 8 und 9, **dadurch gekennzeichnet, dass** als komplexbildende Verbindung Pyridin-2-carbonsäure eingesetzt wird.

11. Verfahren zur Herstellung von 2-(Triazinylcarbonyl)sulfonaniliden der Formel (1-1) nach einem der Ansprüche 9 und 10, **dadurch gekennzeichnet, dass** als Katalysator Eisen(II)sulfat zusammen mit Pyridin-2-carbonsäure eingesetzt wird.

12. Verfahren zur Herstellung von 2-(Triazinylcarbonyl)sulfonaniliden der Formel (1-1) nach einem der Ansprüche 1 bis 11, **dadurch gekennzeichnet, dass** als Lösungsmittel eine Mischung bestehend aus mindestens einem mit Wasser mischbaren organischen Lösungsmitteln und Wasser eingesetzt wird.

13. Verfahren zur Herstellung von 2-(Triazinylcarbonyl)sulfonaniliden der Formel (1-1) nach Anspruch 12, **dadurch gekennzeichnet, dass** als organische Lösungsmittel Acetonitril und/oder 2-Propanol eingesetzt werden.

14. Verfahren zur Herstellung von 2-(Triazinylcarbonyl)sulfonaniliden der Formel (1-1) nach einem der Ansprüche 1 bis 13, **dadurch gekennzeichnet, dass** als Base
- Kaliumcarbonat, Natriumcarbonat oder Cäsiumcarbonat, sowie
- Kaliumhydrogencarbonat oder Natriumhydrogencarbonat, sowie
- Lithiumhydroxid, Natriumhydroxid, Kaliumhydroxid oder Bariumhydroxid , sowie
- Kaliumphosphat (K₃PO₄), Kaliumhydrogenphosphat (K₂HPO₄) oder Natriumphosphat,
oder
- Mischungen aus mindestens zwei der vorgenannten Basen eingesetzt werden.

15. Verfahren zur Herstellung von 2-(Triazinylcarbonyl)sulfonaniliden der Formel (1-1) nach Anspruch 14, **dadurch gekennzeichnet, dass** als Base Kaliumhydrogencarbonat oder Kaliumcarbonat oder ein Gemisch aus beiden eingesetzt wird.

16. Verwendung von N-sulfonylsubstituierten 3-Triazinyloxindolen der Formel (2-1) 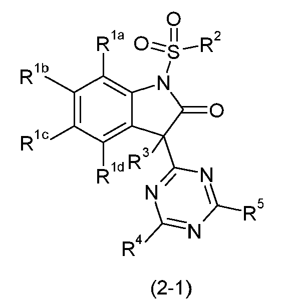 oder deren Salze (2-1 a) 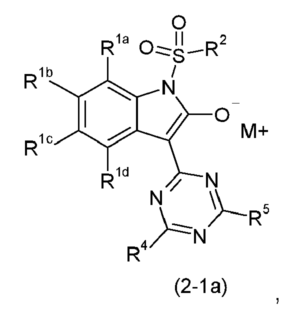 worin jeweils
R^{1a} bis R^{1d} unabhängig voneinander ausgewählt sind aus der Gruppe bestehend aus
Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
(C₁-C₆)-Alkyl, wobei der Alkylrest unsubstituiert ist oder ganz oder teilweise mit Fluor substituiert ist, oder
(C₃-C₇)-Cycloalkyl, wobei der Cycloalkylrest unsubstituiert ist oder ganz oder teilweise mit Fluor substituiert ist,
steht, und
R³ für Wasserstoff steht, und
R⁴ und R⁵ unabhängig voneinander jeweils für Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, (C₁-C₆)-Alkoxy, wobei der Alkoxyrest verzweigt oder unverzweigt ist und
unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
wobei in den Salzen der allgemeinen Formel (2-1 a) M für Li, Na, K, Cs, Ba, Mg, Ca, Zn oder N(R^{c})₄, worin R^{c} = H oder C₁-C₆ Alkyl oder Benzyl ist, steht, und wobei sich die Anzahl der Gegenionen M⁺ nach der jeweiligen Ladung richtet, so dass Verbindung der allgemeinen Formel (2-1 a) insgesamt neutral sind,
als Zwischenprodukte für die Synthese von Wirkstoffen im Bereich der Landwirtschaft.

17. Verfahren zur Herstellung von N-Alkyl-N-[2-(1,3,5-triazin-2-ylcarbonyl)phenyl]alkansulfonamiden der Formel (4-1) 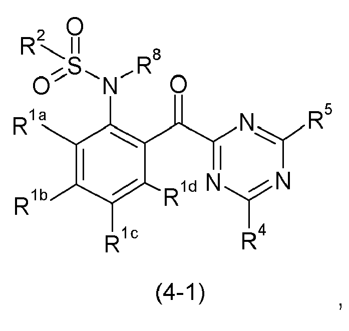 worin
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
(C₁-C₆)-Alkyl, wobei der Alkylrest unsubstituiert ist oder ganz oder teilweise mit Fluor substituiert ist, oder
(C₃-C₇)-Cycloalkyl, wobei der Cycloalkylrest unsubstituiert ist oder ganz oder teilweise mit Fluor substituiert ist,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen, und
R⁸ für
(C₁-C₆)-Alkyl, wobei der Alkylrest unsubstituiert ist oder ganz oder teilweise mit Fluor substituiert ist,
(C₁-C₆)-Cycloalkyl, (C₁-C₆)-Alkenyl oder (C₁-C₆)-Alkoxyalkyl, wobei jeder der genannten Reste unsubstituiert ist oder ganz oder teilweise mit Fluor substituiert ist,
steht, wobei
ein 1,3-Dihydro-2H-indol-2-on der Formel (6-1) 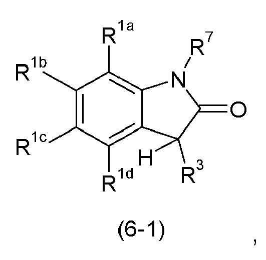 worin
R^{1a} bis R^{1d} wie für Formel (4-1) definiert sind,
R³ für Wasserstoff steht, und
R⁷ für Wasserstoff steht, in einem
ersten Schritt durch
- Arylierung zu einem triazinylsubstituierten Oxindol der Formel (5-1) 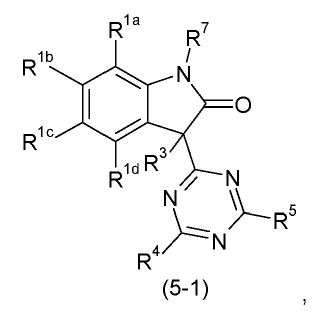 worin
R^{1a} bis R^{1d} und R⁴ und R⁵ wie für die Formel (4-1) und R³ und R⁷ wie für die Formel (6-1) definiert sind,
umgesetzt wird, und die Arylierungsprodukte der Formel (5-1) in einem
zweiten Schritt durch
- Sulfonylierung zu N-sulfonylsubstituierten 3-Triazinyloxindolen der Formel (2-1) 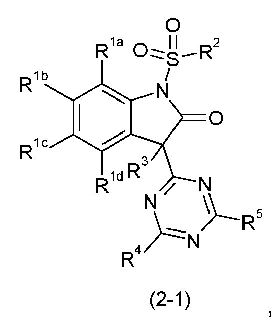 worin
R^{1a} bis R^{1d}, R² sowie R⁴ und R⁵ wie in Formel (4-1) und R³ wie für die Formel (6-1) definiert sind,
umgesetzt werden, und die Sulfonylierungsprodukte der Formel (2-1) in einem
dritten Schritt durch
- oxidative Ringöffnung zu einem 2-(Triazinylcarbonyl)sulfonanilid der Formel (1-1) 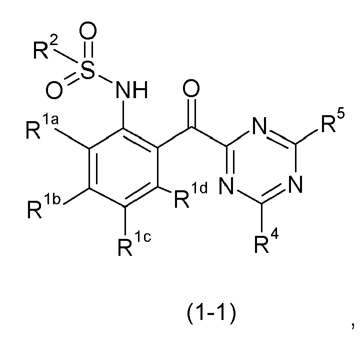 worin
R^{1a} bis R^{1d}, R² sowie R⁴ und R⁵ wie für Formel (4-1) definiert sind,
umgesetzt werden, und die Oxidationsprodukte der Formel (1-1) in einem
vierten Schritt durch
- Alkylierung zu einem N-Alkyl-N-[2-(1,3,5-triazin-2-ylcarbonyl)phenyl] alkansulfonamid der Formel (4-1) 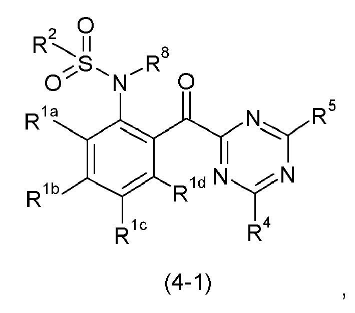 worin
R^{1a} bis R^{1d}, R², R⁴, R⁵ und R⁸ wie für Formel (4-1) definiert sind,
umgesetzt wird, wobei als Alkylierungsreagenz
- X- R⁸, wobei X für Chlor, Brom oder Iod steht und R⁸ wie oben für Formel (4-1) definiert ist, oder
- (R⁸)₂SO₄, worin R⁸ wie oben für Formel (4-1) definiert ist, eingesetzt werden.

18. Verfahren zur Herstellung von N-Alkyl-N-[2-(1,3,5-triazin-2-ylcarbonyl)phenyl] alkansulfonamiden der Formel (4-1) nach Anspruch 17, **dadurch gekennzeichnet, dass** die als Edukt eingesetzten Verbindungen der Formel (6-1) in einem vorhergehenden Verfahrensschritt ausgehend von einem 3-(Alkylsulfanyl)-1,3-dihydro-2H-indol-2-on der Formel (7-1), 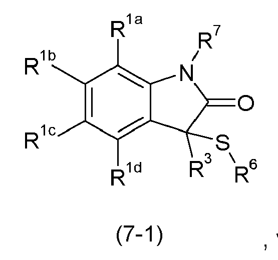 worin
R^{1a} bis R^{1d} wie für Formel (4-1) definiert sind,
R³ für Wasserstoff steht,
R⁷ für Wasserstoff steht, und
R⁶ ein unsubstituiertes oder substituiertes (C₁-C₁₄)-Alkyl, (C₃-C₇)-Cycloalkyl, Benzyl oder ein CH₂-C(O)O-(C₁-C₆)-Alkyl ist,
durch
- Reduktion zu einem 1,3-Dihydro-2H-indol-2-on (6-1) 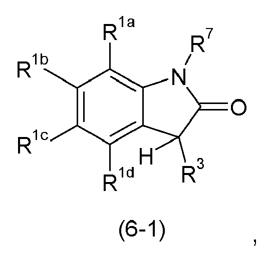 worin
R^{1a} bis R^{1d}, R³ und R⁷ wie für Formel (7-1) definiert sind, umgesetzt wird.

## Claims

1. A process for preparing 2-(triazinylcarbonyl)sulfonanilides of the formula (1-1) 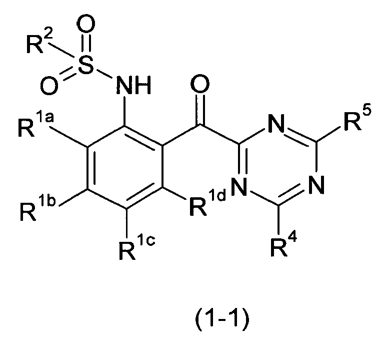 in which
R^{1a} to R^{1d} are each independently selected from the group consisting of
hydrogen, fluorine, chlorine, bromine, iodine and from
(C₁-C₆)-alkyl where the alkyl radical is branched or unbranched and is unsubstituted or substituted by one or more substituents selected from the group consisting of fluorine, chlorine, (C₁-C₄)-alkoxy and (C₃-C₇)-cycloalkyl,
(C₃-C₇)-cycloalkyl where the cycloalkyl radical is unsubstituted or substituted by one or more substituents selected from the group consisting of fluorine, chlorine, (C₁-C₄)-alkyl and (C₃-C₇)-cycloalkyl and (C₁-C₄)-alkoxy,
(C₁-C₆)-alkoxy where the alkoxy radical is branched or unbranched and is unsubstituted or substituted by one or more substituents selected from the group consisting of fluorine, chlorine, (C₁-C₄)-alkoxy and (C₃-C₇)-cycloalkyl,
(C₃-C₇)-cycloalkoxy where the cycloalkoxy radical is unsubstituted or substituted by one or more substituents selected from the group consisting of fluorine, chlorine, (C₁-C₄)-alkyl and (C₁-C₄)-alkoxy,
(C₁-C₆)-alkylthio where the alkylthio radical is branched or unbranched and is unsubstituted or substituted by one or more substituents selected from the group consisting of fluorine, chlorine, (C₁-C₄)-alkyl and (C₁-C₄)-alkoxy,
(C₃-C₇)-cycloalkylthio where the cycloalkylthio radical is unsubstituted or substituted by one or more substituents selected from the group consisting of fluorine, chlorine, (C₁-C₄)-alkyl and (C₁-C₄)-alkoxy, and
phenyl or 1-naphthyl or 2-naphthyl or a five- or six-membered heteroaromatic ring having 1 to 2 heteroatoms, where the heteroatoms are each selected independently from the group consisting of O and N, and where the aryl or heteroaryl radical is unsubstituted or substituted by one or more substituents selected from the group consisting of fluorine, chlorine, bromine, iodine, (C₁-C₄) -alkyl, (C₁-C₄)-alkoxy and (C₃-C₇)-cycloalkyl and (C₁-C₄)-alkylthio, and
R² is
(C₁-C₆)-alkyl where the alkyl radical is unsubstituted or fully or partly substituted by fluorine, or
(C₃-C₇)-cycloalkyl where the cycloalkyl radical is unsubstituted or fully or partly substituted by fluorine,
and
R⁴ and R⁵ are each hydrogen,
(C₁-C₆)-alkyl where the alkyl radical is unsubstituted or substituted by one or more substituents selected from fluorine, chlorine, (C₁-C₄)-alkoxy and (C₃-C₇)-cycloalkyl,or
(C₁-C₆)-alkoxy where the alkoxy radical is branched or unbranched and is unsubstituted or substituted by one or more substituents selected from the group consisting of fluorine, chlorine, (C₁-C₄)-alkoxy and (C₃-C₇)-cycloalkyl,
which comprises
- using as the reactant an N-sulfonyl-substituted 3-triazinyloxindole of the formula (2-1) 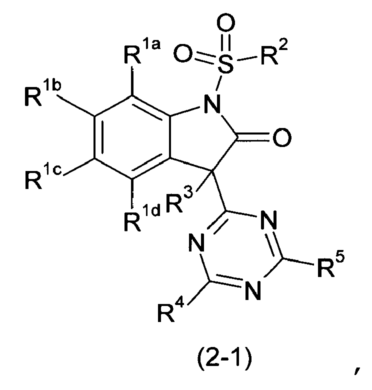 in which
R^{1a} to R^{1d} and R², R⁴ and R⁵ are each as defined in formula (1-1), and
R³ is hydrogen
and
- converting the reactant of the general formula (2-1) initially charged in a solvent in the presence
- of a base and
- of an oxidizing agent.

2. The process for preparing 2-(triazinylcarbonyl)sulfonanilides of the formula (1-1) as claimed in claim 1, where
R^{1a} to R^{1d} are each independently selected from the group consisting of hydrogen, fluorine, chlorine, bromine, iodine and from
(C₁-C₆)-alkyl where the alkyl radical is branched or unbranched and is unsubstituted or substituted by one or more substituents selected from the group consisting of fluorine, chlorine, (C₁-C₄)-alkoxy or (C₃-C₇)-cycloalkyl,
(C₃-C₇)-cycloalkyl where the cycloalkyl radical is unsubstituted or substituted by one or more substituents selected from the group consisting of fluorine, chlorine, (C₁-C₄)-alkyl or (C₃-C₇)-cycloalkyl or (C₁-C₄)-alkoxy,
(C₁-C₆)-alkoxy where the alkoxy radical is branched or unbranched and is unsubstituted or substituted by one or more substituents selected from the group consisting of fluorine, chlorine, (C₁-C₄)-alkoxy or (C₃-C₇)-cycloalkyl,
(C₃-C₇)-cycloalkoxy where the cycloalkoxy radical is unsubstituted or substituted by one or more substituents selected from the group consisting of fluorine, chlorine, (C₁-C₄)-alkyl or (C₁-C₄)-alkoxy,
and
R² is (C₁-C₆)-alkyl where the alkyl radical is fully or partly substituted by fluorine, and
R³ is as defined in claim 1, and
R⁴ and R⁵ are each independently
(C₁-C₆)-alkoxy where the alkoxy radical is branched or unbranched and is unsubstituted or substituted by one or more substituents selected from the group consisting of fluorine, chlorine, (C₁-C₉)-alkoxy or (C₃-C₇)-cycloalkyl.

3. The process for preparing 2-(triazinylcarbonyl)sulfonanilides of the formula (1-1) as claimed in either of claims 1 and 2, where
R^{1a} to R^{1d} are each independently selected from the group
consisting of hydrogen, fluorine, chlorine, methoxy, and
R² is difluoromethyl and R³ is hydrogen, and
R⁴ and R⁵ are each methoxy.

4. The process for preparing 2-(triazinylcarbonyl)sulfonanilides of the formula (1-1) as claimed in any of claims 1 to 3, wherein the oxidizing agent is used in combination with a catalyst which has at least one heavy metal or the salt of a heavy metal as a constituent.

5. The process for preparing 2-(triazinylcarbonyl)sulfonanilides of the formula (1-1) as claimed in claim 4, wherein the oxidizing agent used is hydrogen peroxide in conjunction with a catalyst which has at least one heavy metal or the salt of a heavy metal as a constituent.

6. The process for preparing 2-(triazinylcarbonyl)sulfonanilides of the formula (1-1) as claimed in claim 4, wherein the oxidizing agent used is potassium permanganate without or with a catalyst which has at least one heavy metal or the salt of a heavy metal as a constituent.

7. The process for preparing 2-(triazinylcarbonyl)sulfonanilides of the formula (1-1) as claimed in any of claims 1 to 6, wherein the catalyst used comprises:
- iron salts, iron powder, copper salts or copper powder, or
- mixtures of at least two of the aforementioned catalysts.

8. The process for preparing 2-(triazinylcarbonyl)sulfonanilides of the formula (1-1) as claimed in any of claims 4 to 7, wherein the catalyst used comprises:
- one or more iron salts together with complexing compounds, or
- one or more copper salts together with complexing compounds, or
- mixtures of at least two of the aforementioned catalyst systems.

9. The process for preparing 2-(triazinylcarbonyl)sulfonanilides of the formula (1-1) as claimed in either of claims 7 and 8, wherein
- the iron salts used are iron(II) sulfate and/or iron chloride, and
- the copper salts used are copper(II) sulfate and/or copper(II) chloride.

10. The process for preparing 2-(triazinylcarbonyl)sulfonanilides of the formula (1-1) as claimed in either of claims 8 and 9, wherein the complexing compound used is pyridine-2-carboxylic acid.

11. The process for preparing 2-(triazinylcarbonyl)sulfonanilides of the formula (1-1) as claimed in either of claims 9 and 10, wherein the catalyst used is iron(II) sulfate together with pyridine-2-carboxylic acid.

12. The process for preparing 2-(triazinylcarbonyl)sulfonanilides of the formula (1-1) as claimed in any of claims 1 to 11, wherein the solvent used is a mixture consisting of at least one water-miscible organic solvent and water.

13. The process for preparing 2-(triazinylcarbonyl)sulfonanilides of the formula (1-1) as claimed in claim 12, wherein the organic solvents used are acetonitrile and/or 2-propanol.

14. The process for preparing 2-(triazinylcarbonyl)sulfonanilides of the formula (1-1) as claimed in any of claims 1 to 13, wherein the base used is
- potassium carbonate, sodium carbonate or cesium carbonate, and
- potassium hydrogencarbonate or sodium hydrogencarbonate, and
- lithium hydroxide, sodium hydroxide, potassium hydroxide or barium hydroxide, and
potassium phosphate (K₃PO₄), potassium hydrogenphosphate (K₂HPO₄) or sodium phosphate,
or
- mixtures of at least two of the aforementioned bases.

15. The process for preparing 2-(triazinylcarbonyl)sulfonanilides of the formula (1-1) as claimed in claim 14, wherein the base used is potassium hydrogencarbonate or potassium carbonate or a mixture of the two.

16. The use of N-sulfonyl-substituted 3-triazinyloxindoles of the formula (2-1) 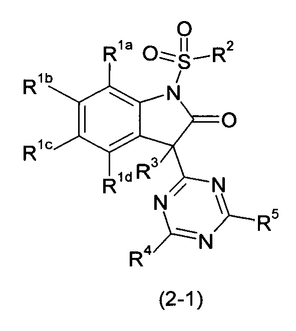 or salts thereof (2-1a) 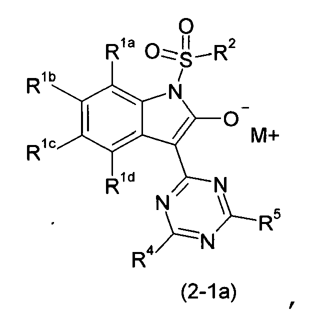 in each of which
R^{1a} to R^{1d} are each independently selected from the group consisting of
hydrogen, fluorine, chlorine, bromine, iodine and from
(C₁-C₆)-alkyl where the alkyl radical is branched or unbranched and is unsubstituted or substituted by one or more substituents selected from the group consisting of fluorine, chlorine, (C₁-C₄)-alkoxy and (C₃-C₇)-cycloalkyl,
(C₃-C₇)-cycloalkyl where the cycloalkyl radical is unsubstituted or substituted by one or more substituents selected from the group consisting of fluorine, chlorine, (C₁-C₄)-alkyl and (C₃-C₇)-cycloalkyl and (C₁-C₄)-alkoxy,
(C₁-C₆)-alkoxy where the alkoxy radical is branched or unbranched and is unsubstituted or substituted by one or more substituents selected from the group consisting of fluorine, chlorine, (C₁-C₄)-alkoxy and (C₃-C₇)-cycloalkyl,
(C₃-C₇)-cycloalkoxy where the cycloalkoxy radical is unsubstituted or substituted by one or more substituents selected from the group consisting of fluorine, chlorine, (C₁-C₄)-alkyl and (C₁-C₄)-alkoxy,
(C₁-C₆)-alkylthio where the alkylthio radical is branched or unbranched and is unsubstituted or substituted by one or more substituents selected from the group consisting of fluorine, chlorine, (C₁-C₄) -alkyl and (C₁-C₄) -alkoxy,
(C₃-C₇)-cycloalkylthio where the cycloalkylthio radical is unsubstituted or substituted by one or more substituents selected from the group consisting of fluorine, chlorine, (C₁-C₄) -alkyl and (C₁-C₄)-alkoxy and
phenyl or 1-naphthyl or 2-naphthyl or a five- or six-membered heteroaromatic ring having 1 to 2 heteroatoms, where the heteroatoms are each selected independently from the group consisting of O and N, and where the aryl or heteroaryl radical is unsubstituted or substituted by one or more substituents selected from the group consisting of fluorine, chlorine, bromine, iodine, (C₁-C₄)-alkyl, (C₁-C₄)-alkoxy and (C₃-C₇)-cycloalkyl and (C₁-C₄)-alkylthio, and
R² is
(C₁-C₆)-alkyl where the alkyl radical is unsubstituted or fully or partly substituted by fluorine, or
(C₃-C₇)-cycloalkyl where the cycloalkyl radical is unsubstituted or fully or partly substituted by fluorine,
R³ is hydrogen, and
R⁴ and R⁵ are each hydrogen,
(C₁-C₆)-alkyl where the alkyl radical is unsubstituted or substituted by one or more substituents selected from the group consisting of fluorine, chlorine, (C₁-C₄)-alkoxy or (C₃-C₇)-cycloalkyl,
(C₁-C₆)-alkoxy where the alkoxy radical is branched or unbranched and is unsubstituted or substituted by one or more substituents selected from the group consisting of fluorine, chlorine, (C₁-C₄)-alkoxy and (C₃-C₇)-cycloalkyl,
wherein M in the salts of the general formula (2-1a) is Li, Na, K, Cs, Ba, Mg, Ca, Zn or N(R^{c})₄ in which R^{c} = H or (C₁-C₆)-alkyl or benzyl, and
where the number of counterions M⁺ is guided by the particular charge, such that the compound of the general formula (2-1a) is uncharged overall,
as intermediates for the synthesis of active ingredients in the agricultural sector.

17. A process for preparing N-alkyl-N-[2-(1,3,5-triazin-2-ylcarbonyl)phenyl]alkanesulfonamides of the formula (4-1) 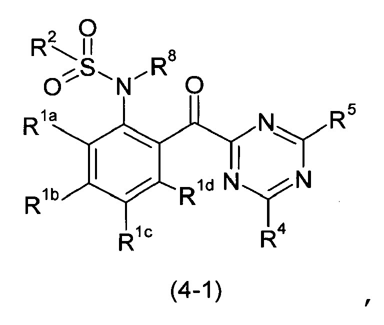 in which
R^{1a} to R^{1d} are each independently selected from the group consisting of
hydrogen, fluorine, chlorine, bromine, iodine and from
(C₁-C₆)-alkyl where the alkyl radical is unsubstituted or substituted by one or more substituents selected from the group consisting of fluorine, chlorine, (C₁-C₄)-alkoxy and (C₃-C₇)-cycloalkyl,
(C₃-C₇)-cycloalkyl where the cycloalkyl radical is unsubstituted or substituted by one or more substituents selected from the group consisting of fluorine, chlorine, (C₁-C₄)-alkyl and (C₃-C₇) cycloalkyl and (C₁-C₄)-alkoxy,
(C₁-C₆)-alkoxy where the alkoxy radical is unsubstituted or substituted by one or more substituents selected from the group consisting of fluorine, chlorine, (C₁-C₄)-alkoxy and (C₃-C₇)-cycloalkyl,
(C₃-C₇)-cycloalkoxy where the cycloalkoxy radical is unsubstituted or substituted by one or more substituents selected from the group consisting of fluorine, chlorine, (C₁-C₄)-alkyl and (C₁-C₄) -alkoxy,
(C₁-C₆)-alkylthio where the alkylthio radical is unsubstituted or substituted by one or more substituents selected from the group consisting of fluorine, chlorine, (C₁-C₄)-alkyl and (C₁-C₄) -alkoxy,
(C₃-C₇)-cycloalkylthio where the cycloalkylthio radical is unsubstituted or substituted by one or more substituents selected from the group consisting of fluorine, chlorine, (C₁-C₄)-alkyl and (C₁-C₄)-alkoxy, and
phenyl or 1-naphthyl or 2-naphthyl or a five- or six-membered heteroaromatic ring having 1 to 2 heteroatoms, where the heteroatoms are each selected independently from the group consisting of O and N, and where the aryl or heteroaryl radical is unsubstituted or substituted by one or more substituents selected from the group consisting of fluorine, chlorine, bromine, iodine, (C₁-C₄)-alkyl, (C₁-C₄) -alkoxy and (C₃-C₇)-cycloalkyl and (C₁-C₄)-alkylthio, and
R2 is
(C₁-C₆)-alkyl where the alkyl radical is unsubstituted or fully or partly substituted by fluorine, or
(C₃-C₇)-cycloalkyl where the cycloalkyl radical is unsubstituted or fully or partly substituted by fluorine,
R⁴ and R⁵ are each independently
hydrogen,
(C₁-C₆)-alkyl where the alkyl radical is unsubstituted or substituted by one or more substituents selected from the group consisting of fluorine, chlorine, (C₁-C₄)-alkoxy and (C₃-C₇)-cycloalkyl,
(C₁-C₆)-alkoxy where the alkoxy radical is branched or unbranched and is unsubstituted or substituted by one or more substituents selected from the group consisting of fluorine, chlorine, (C₁-C₄)-alkoxy and (C₃-C₇)-cycloalkyl,
and
R8 is
(C₁-C₆)-alkyl where the alkyl radical is unsubstituted or partly or fully substituted by fluorine,
(C₁-C₆) -cycloalkyl, (C₁-C₆) -alkenyl or (C₁-C₆) - alkoxyalkyl, where each of the radicals mentioned is unsubstituted or substituted partly or fully by fluorine,
wherein
a 1,3-dihydro-2H-indol-2-one of the formula (6-1) 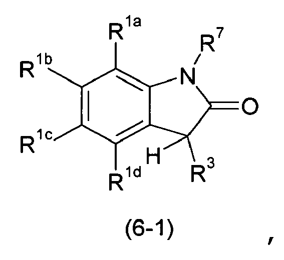 in which
R^{1a} to R^{1d} are each as defined for formula (4-1),
R³ is hydrogen, and
R⁷ is hydrogen, is converted in a
first step by
- arylation to give a triazinyl-substituted oxindole of the formula (5-1) 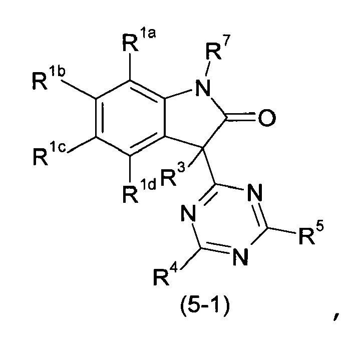 in which
R^{1a} to R^{1d} and R⁴ and R⁵ are each as defined for the formula (4-1) and R³ and R⁷ are each as defined for the formula (6-1),
and the arylation products of the formula (5-1) are converted in a
second step by
- sulfonylation to give N-sulfonyl-substituted 3-triazinyloxindoles of the formula (2-1) 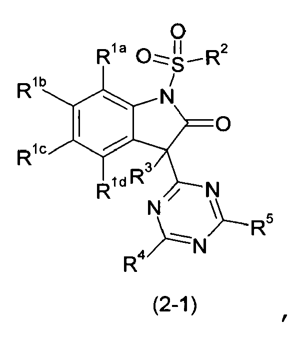 in which
R^{1a} to R^{1d}, R² and R⁴ and R⁵ are each as defined in formula (4-1) and R³ is as defined for formula (6-1),
and the sulfonylation products of the formula (2-1) are converted in a
third step by
- oxidative ring opening to give a 2-(triazinylcarbonyl)sulfonanilide of the formula (1-1) 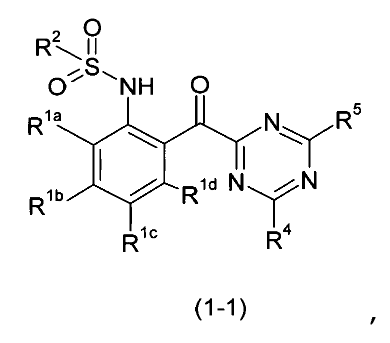 in which
R^{1a} to R^{1d}, R² and R⁴ and R⁵ are each as defined for formula (4-1),
and the oxidation products of the formula (1-1) are converted in a
fourth step by
- alkylation to give an N-alkyl-N-[2-(1,3,5-triazin-2-ylcarbonyl)phenyl] alkanesulfonamide of the formula (4-1) 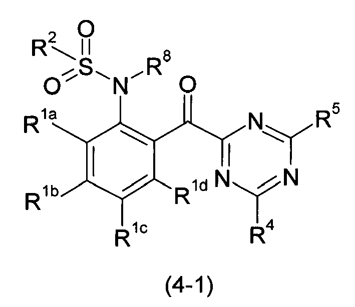 in which
R^{1a} to R^{1d}, R², R⁴, R⁵ and R⁸ are each as defined for formula (4-1),
wherein the alkylating reagent used is
- X- R⁸ where X is chlorine, bromine or iodine and R⁸ is as defined above for formula (4-1), or
- (R⁸)₂SO₄ in which R⁸ is as defined above for formula (4-1).

18. The process for preparing N-alkyl-N-[2-(1,3,5-triazin-2-ylcarbonyl)phenyl] alkanesulfonamides of the formula (4-1) as claimed in claim 17, wherein the compounds of the formula (6-1) used as the reactant are converted in a preceding process step proceeding from a 3-(alkylsulfanyl)-1,3-dihydro-2H-indol-2-one of the formula (7-1) 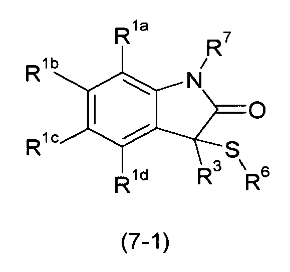 in which
R^{1a} to R^{1d} are each as defined for formula (4-1),
R³ is hydrogen,
R⁷ is hydrogen, and
R⁶ is an unsubstituted or substituted (C₁-C₁₄)-alkyl, (C₃-C₇)-cycloalkyl, benzyl or a CH₂-C(O)O-(C₁-C₆)-alkyl,
by
- reduction to give a 1,3-dihydro-2H-indol-2-one (6-1) 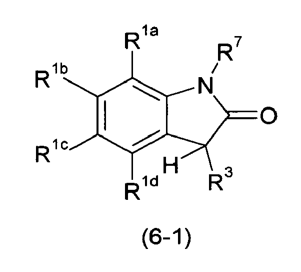 in which
R^{1a} to R^{1d}, R³ and R⁷ are each as defined for formula (7-1).

## Revendications

1. Procédé de préparation de 2-(triazinylcarbonyl)sulfonanilides de formule (1-1) 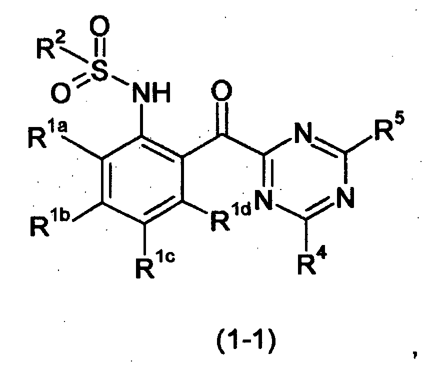 dans laquelle
R^{1a} à R^{1d} sont chacun indépendamment de l'autre choisis dans le groupe consistant en l'atome d'hydrogène, le fluor, le chlore, le brome, l'iode, ainsi que
les groupes alkyle en C₁-C₆, le radical alkyle ayant une chaîne droite ou ramifiée et étant non substitué, ou étant substitué par un ou plusieurs substituants choisis dans le groupe consistant en les radicaux fluoro, chloro, alcoxy en C₁-C₄ ou cycloalkyle en C₃-C₇,
les groupes cycloalkyle en C₃-C₇, le radical cycloalkyle étant non substitué, ou substitué par un ou plusieurs substituants choisis dans le groupe consistant en les radicaux fluoro, chloro, alkyle en C₁-C₄ ou cycloalkyle en C₃-C₇ ou alcoxy en C₁-C₄,
les groupes alcoxy en C₁-C₆, le radical alcoxy ayant une chaîne droite ou ramifiée et étant non substitué, ou substitué par un ou plusieurs substituants choisis dans le groupe consistant en les radicaux fluoro, chloro, alcoxy en C₁-C₄ ou cycloalkyle en C₃-C₇,
les groupes cycloalcoxy en C₃-C₇, le radical cycloalkyle étant non substitué, ou substitué par un ou plusieurs substituants choisis dans le groupe consistant en les radicaux fluoro, chloro, alkyle en C₁-C₄ ou alcoxy en C₁-C₄,
les groupes alkylthio en C₁-C₆, le radical alkylthio ayant une chaîne droite ou ramifiée et étant non substitué, ou substitué par un ou plusieurs substituants choisis dans le groupe consistant en les radicaux fluoro, chloro, alkyle en C₁-C₄ ou alcoxy en C₁-C₄,
les groupes cycloalkylthio en C₃-C₇, le radical cycloalkylthio étant non substitué, ou substitué par un ou plusieurs substituants choisis dans le groupe consistant en les radicaux fluoro, chloro, alkyle en C₁-C₄ ou alcoxy en C₁-C₄, et
le groupe phényle ou 1-naphtyle, ou 2-naphtyle, ou un noyau hétéroaromatique à cinq ou six chaînons ayant 1 à 2 hétéroatomes, les hétéroatomes étant choisis indépendamment l'un de l'autre dans le groupe consistant en O ou N, et le radical aryle ou hétéroaryle étant non substitué, ou substitué par un ou plusieurs substituants choisis dans le groupe consistant en les radicaux fluoro, chloro, bromo, iodo, alkyle en C₁-C₄, alcoxy en C₁-C₄, cycloalkyle en C₃-C₇ ou alkylthiol en C₁-C₄, et
R² représente
un groupe alkyle en C₁-C₆, le radical alkyle étant non substitué, ou en totalité ou en partie substitué par un ou deux radicaux fluoro, ou
un groupe cycloalkyle en C₃-C₇, le radical cycloalkyle étant non substitué, ou en totalité ou en partie substitué par un ou des radicaux fluoro,
et
R⁴ et R⁵ représentent chacun un atome d'hydrogène, un groupe alkyle en C₁-C₆, le radical alkyle étant non substitué, ou substitué par un ou plusieurs substituants choisis dans le groupe consistant en les radicaux fluoro, chloro, alcoxy en C₁-C₄ ou cycloalkyle en C₃-C₇, ou
un groupe alcoxy en C₁-C₆, le radical alcoxy ayant une chaîne droite ou ramifiée, et étant non substitué, ou substitué par un ou plusieurs substituants choisis dans le groupe consistant en les radicaux fluoro, chloro, alcoxy en C₁-C₄ ou cycloalkyle en C₃-C₇,
**caractérisé en ce que**
- on utilise en tant que matière de départ un 3-triazinyloxindole à substitution N-sulfonyle de formule (2-1) 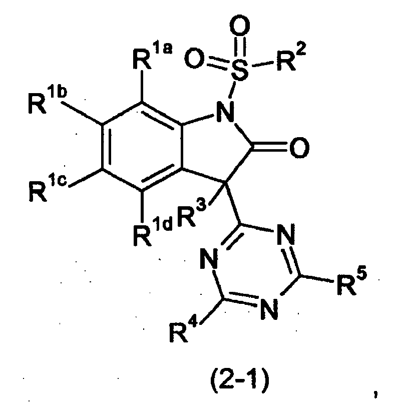 dans laquelle
R^{1a} à R^{1d}, ainsi que R², R⁴ et R⁵, sont chacun définis comme dans la formule (1-1), et
R³ est un atome d'hydrogène,
et **en ce qu'**on fait réagir la matière de départ, placée dans un solvant, de formule générale (2-1), en présence
- d'une base et
- d'un oxydant.

2. Procédé de préparation de 2-(triazinylcarbonyl)sulfonanilides de formule (1-1) selon la revendication 1, dans lequel
R^{1a} à R^{1d} sont chacun indépendamment de l'autre choisis dans le groupe consistant en l'atome d'hydrogène, le fluor, le chlore, le brome, l'iode, ainsi que
les groupes alkyle en C₁-C₆, le radical alkyle ayant une chaîne droite ou ramifiée et étant non substitué, ou étant substitué par un ou plusieurs substituants choisis dans le groupe consistant en les radicaux fluoro, chloro, alcoxy en C₁-C₄ ou cycloalkyle en C₃-C₇,
les groupes cycloalkyle en C₃-C₇, le radical cycloalkyle étant non substitué, ou substitué par un ou plusieurs substituants choisis dans le groupe consistant en les radicaux fluoro, chloro, alkyle en C₁-C₄ ou cycloalkyle en C₃-C₇ ou alcoxy en C₁-C₄,
les groupes alcoxy en C₁-C₆, le radical alcoxy ayant une chaîne droite ou ramifiée et étant non substitué, ou substitué par un ou plusieurs substituants choisis dans le groupe consistant en les radicaux fluoro, chloro, alcoxy en C₁-C₄ ou cycloalkyle en C₃-C₇,
les groupes cycloalcoxy en C₃-C₇, le radical cycloalkyle étant non substitué, ou substitué par un ou plusieurs substituants choisis dans le groupe consistant en les radicaux fluoro, chloro, alkyle en C₁-C₄ ou alcoxy en C₁-C₄, et
R² représente un groupe alkyle en C₁-C₆, le radical alkyle étant en totalité ou en partie substitué par un ou des radicaux fluoro, et
R³ est tel que défini dans la revendication 1, et
R⁴ et R⁵ représentent chacun indépendamment l'un de l'autre
un groupe alcoxy en C₁-C₆, le radical alcoxy ayant une chaîne droite ou ramifiée, et étant non substitué, ou substitué par un ou plusieurs substituants choisis dans le groupe consistant en les radicaux fluoro, chloro, alcoxy en C₁-C₄ ou cycloalkyle en C₃-C₇.

3. Procédé de préparation de 2-(triazinylcarbonyl)sulfonanilides de formule (1-1) selon l'une des revendications 1 ou 2, dans lequel
R^{1a} à R^{1d} sont chacun indépendamment de l'autre choisis dans le groupe consistant en l'atome d'hydrogène, les groupes fluoro, chloro, méthoxy, et
R² est une groupe difluorométhyle et R³ est un atome d'hydrogène, et
R⁴ et R⁵ représentent chacun un groupe méthoxy.

4. Procédé de préparation de 2-(triazinylcarbonyl)sulfonanilides de formule (1-1) selon l'une des revendications 1 à 3, **caractérisé en ce que** l'oxydant est utilisé en combinaison avec un catalyseur qui en tant que constituant comprend au moins un métal lourd ou le sel d'un métal lourd.

5. Procédé de préparation de 2-(triazinylcarbonyl)sulfonanilides de formule (1-1) selon la revendication 4, **caractérisé en ce qu'**on utilise en tant qu'oxydant du peroxyde d'hydrogène en même temps qu'un catalyseur, qui comprend en tant que constituant au moins un métal lourd ou le sel d'un métal lourd.

6. Procédé de préparation de 2-(triazinylcarbonyl)sulfonanilides de formule (1-1) selon la revendication 4, **caractérisé en ce qu'**on utilise en tant qu'oxydant du permanganate de potassium, sans catalyseur ou avec un catalyseur, qui en tant que constituant comprend au moins un métal lourd ou le sel d'un métal lourd.

7. Procédé de préparation de 2-(triazinylcarbonyl)sulfonanilides de formule (1-1) selon l'une des revendications 1 à 6, **caractérisé en ce qu'**on utilise en tant que catalyseur
- des sels de fer, de la poudre de fer, des sels de cuivre ou de la poudre de cuivre, ou
- des mélanges d'au moins deux des catalyseurs mentionnés ci-dessus.

8. Procédé de préparation de 2-(triazinylcarbonyl)sulfonanilides de formule (1-1) selon l'une des revendications 4 à 7, **caractérisé en ce qu'**on utilise en tant que catalyseur
- un ou plusieurs sels de fer, en même temps que des composés complexants, ou
- un ou plusieurs sels de cuivre, en même temps que des composés complexants, ou
- des mélanges d'au moins deux des systèmes catalyseurs mentionnés ci-dessus.

9. Procédé de préparation de 2-(triazinylcarbonyl)sulfonanilides de formule (1-1) selon l'une des revendications 7 et 8, **caractérisé en ce qu'**on utilise
- en tant que sels de fer, du sulfate de fer (II) et/ou du chlorure de fer, et
- en tant que sels de cuivre, du sulfate de cuivre(II) ou du chlorure de cuivre(II).

10. Procédé de préparation de 2-(triazinylcarbonyl)sulfonanilides de formule (1-1) selon l'une des revendications 8 et 9, **caractérisé en ce qu'**on utilise en tant que composé complexant de l'acide pyridine-2-carboxylique.

11. Procédé de préparation de 2-(triazinylcarbonyl)sulfonanilides de formule (1-1) selon l'une des revendications 9 et 10, **caractérisé en ce qu'**on utilise en tant que catalyseur du sulfate de fer(II) en même temps que de l'acide pyridine-2-carboxylique.

12. Procédé de préparation de 2-(triazinylcarbonyl)sulfonanilides de formule (1-1) selon l'une des revendications 1 à 11, **caractérisé en ce qu'**on utilise en tant que solvant un mélange constitué d'au moins un solvant organique miscible à l'eau, et d'eau.

13. Procédé de préparation de 2-(triazinylcarbonyl)sulfonanilides de formule (1-1) selon la revendication 12, **caractérisé en ce qu'**on utilise en tant que solvants organiques de l'acétonitrile et/ou du 2-propanol.

14. Procédé de préparation de 2-(triazinylcarbonyl)sulfonanilides de formule (1-1) selon l'une des revendications 1 à 13, **caractérisé en ce qu'**on utilise en tant que base
- du carbonate de potassium, du carbonate de sodium ou du carbonate de césium, ainsi que
- de l'hydrogénocarbonate de potassium ou de l'hydrogénocarbonate de sodium, ainsi que
- de l'hydroxyde de lithium, de l'hydroxyde de sodium, de l'hydroxyde de potassium ou de l'hydroxyde de baryum, ainsi que
- du phosphate de potassium (K₃PO₄), de l'hydrogénophosphate de potassium (K₂HPO₄) ou du phosphate de sodium, ou
- des mélanges d'au moins deux des bases mentionnées ci-dessus.

15. Procédé de préparation de 2-(triazinylcarbonyl)sulfonanilides de formule (1-1) selon la revendication 14, **caractérisé en ce qu'**on utilise en tant que base de l'hydrogénocarbonate de potassium ou du carbonate de potassium ou un mélange des deux.

16. Utilisation de 3-triazinyloxindoles à substitution N-sulfonyle de formule (2-1) 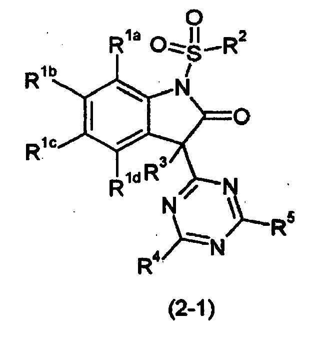 ou de ses sels (2-1a) 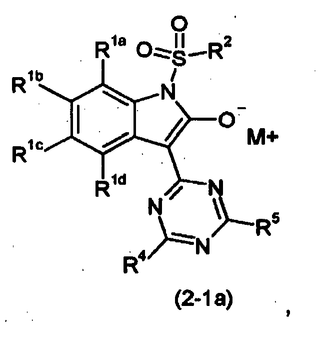 dans chacune desquelles
R^{1a} à R^{1d} sont chacun indépendamment de l'autre choisis dans le groupe consistant en l'atome d'hydrogène, le fluor, le chlore, le brome, l'iode, ainsi que
les groupes alkyle en C₁-C₆, le radical alkyle ayant une chaîne droite ou ramifiée et étant non substitué, ou étant substitué par un ou plusieurs substituants choisis dans le groupe consistant en les radicaux fluoro, chloro, alcoxy en C₁-C₄ ou cycloalkyle en C₃-C₇,
les groupes cycloalkyle en C₃-C₇, le radical cycloalkyle étant non substitué, ou substitué par un ou plusieurs substituants choisis dans le groupe consistant en les radicaux fluoro, chloro, alkyle en C₁-C₄ ou cycloalkyle en C₃-C₇ ou alcoxy en C₁-C₄,
les groupes alcoxy en C₁-C₆, le radical alcoxy ayant une chaîne droite ou ramifiée et étant non substitué, ou substitué par un ou plusieurs substituants choisis dans le groupe consistant en les radicaux fluoro, chloro, alcoxy en C₁-C₄ ou cycloalkyle en C₃-C₇,
les groupes cycloalcoxy en C₃-C₇, le radical cycloalkyle étant non substitué, ou substitué par un ou plusieurs substituants choisis dans le groupe consistant en les radicaux fluoro, chloro, alkyle en C₁-C₄ ou alcoxy en C₁-C₄,
les groupes alkylthio en C₁-C₆, le radical alkylthio ayant une chaîne droite ou ramifiée et étant non substitué, ou substitué par un ou plusieurs substituants choisis dans le groupe consistant en les radicaux fluoro, chloro, alkyle en C₁-C₄ ou alcoxy en C₁-C₄,
les groupes cycloalkylthio en C₃-C₇, le radical cycloalkylthio étant non substitué, ou substitué par un ou plusieurs substituants choisis dans le groupe consistant en les radicaux fluoro, chloro, alkyle en C₁-C₄ ou alcoxy en C₁-C₄, et
le groupe phényle ou 1-naphtyle, ou 2-naphtyle, ou un noyau hétéroaromatique à cinq ou six chaînons ayant 1 à 2 hétéroatomes, les hétéroatomes étant choisis indépendamment l'un de l'autre dans le groupe consistant en O ou N, et le radical aryle ou hétéroaryle étant non substitué, ou substitué par un ou plusieurs substituants choisis dans le groupe consistant en les radicaux fluoro, chloro, bromo, iodo, alkyle en C₁-C₄, alcoxy en C₁-C₄, cycloalkyle en C₃-C₇ ou alkylthiol en C₁-C₄, et
R² représente
un groupe alkyle en C₁-C₆, le radical alkyle étant non substitué, ou en totalité ou en partie substitué par un ou deux radicaux fluoro, ou
un groupe cycloalkyle en C₃-C₇, le radical cycloalkyle étant non substitué, ou substitué en totalité ou en partie par un ou des radicaux fluoro, et R³ est un atome d'hydrogène, et
R⁴ et R⁵ représentent chacun indépendamment de l'autre un atome d'hydrogène,
un groupe alkyle en C₁-C₆, le radical alkyle étant non substitué, ou substitué par un ou plusieurs substituants choisis dans le groupe consistant en les radicaux fluoro, chloro, alcoxy en C₁-C₄ ou cycloalkyle en C₃-C₇,
un groupe alcoxy en C₁-C₆, le radical alcoxy ayant une chaîne droite ou ramifiée, et étant non substitué, ou substitué par un ou plusieurs substituants choisis dans le groupe consistant en les radicaux fluoro, chloro, alcoxy en C₁-C₄ ou cycloalkyle en C₃-C₇,
pour laquelle, dans les sels de formule générale (2-1a), M représente Li, Na, K, Cs, Ba, Mg, Ca, Zn ou N(R^{c})₄, où R^{c} est H ou un groupe alkyle en C₁-C₆ ou benzyle, et
pour laquelle le nombre des contre-ions M⁺ dépend de la charge pour laquelle le composé de formule générale (2-1a) est globalement neutre,
en tant que produits intermédiaires pour la synthèse de matières actives dans le domaine de l'agriculture.

17. Procédé de préparation de N-alkyle-N-[2-(1,3,5-triazin-2-ylcarbonyl)phényl]alcanesulfonamides de formule (4-1) 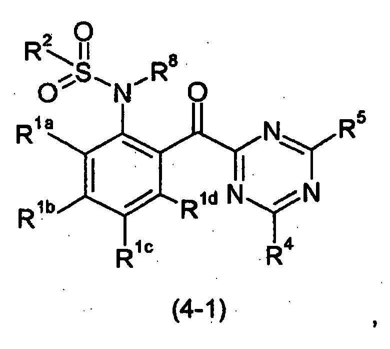 dans laquelle
R^{1a} à R^{1d} sont chacun indépendamment de l'autre choisis dans le groupe consistant en l'atome d'hydrogène, le fluor, le chlore, le brome, l'iode, ainsi que
les groupes alkyle en C₁-C₆, le radical alkyle étant non substitué, ou substitué par un ou plusieurs substituants choisis dans le groupe consistant en les radicaux fluoro, chloro, alcoxy en C₁-C₄ ou cycloalkyle en C₃-C₇,
les groupes cycloalkyle en C₃-C₇, le radical cycloalkyle étant non substitué, ou substitué par un ou plusieurs substituants choisis dans le groupe consistant en les radicaux fluoro, chloro, alkyle en C₁-C₄ ou cycloalkyle en C₃-C₇ ou alcoxy en C₁-C₄,
les groupes alcoxy en C₁-C₆, le radical alcoxy étant non substitué, ou substitué par un ou plusieurs substituants choisis dans le groupe consistant en les radicaux fluoro, chloro, alcoxy en C₁-C₄ ou cycloalkyle en C₃-C₇,
les groupes cycloalcoxy en C₃-C₇, le radical cycloalcoxy étant non substitué, ou étant substitué par un ou plusieurs substituants choisis dans le groupe consistant en les radicaux fluoro, chloro, alkyle en C₁-C₄ ou alcoxy en C₁-C₄,
les groupes alkylthio en C₁-C₆, le radical alkylthio étant non substitué, ou étant substitué par un ou plusieurs substituants choisis dans le groupe consistant en les radicaux fluoro, chloro, alkyle en C₁-C₄ ou alcoxy en C₁-C₄,
les groupes cycloalkylthio en C₃-C₇, le radical cycloalkylthio étant non substitué, ou substitué par un ou plusieurs substituants choisis dans le groupe consistant en les radicaux fluoro, chloro, alkyle en C₁-C₄ ou alcoxy en C₁-C₄, et
le groupe phényle ou 1-naphtyle, ou 2-naphtyle, ou un noyau hétéroaromatique à cinq ou six chaînons ayant 1 à 2 hétéroatomes, les hétéroatomes étant choisis indépendamment l'un de l'autre dans le groupe consistant en O ou N, et le radical aryle ou hétéroaryle étant non substitué, ou substitué par un ou plusieurs substituants choisis dans le groupe consistant en les radicaux fluoro, chloro, bromo, iodo, alkyle en C₁-C₄, alcoxy en C₁-C₄, cycloalkyle en C₃-C₇ ou alkylthiol en C₁-C₄, et
R² représente
un groupe alkyle en C₁-C₆, le radical alkyle étant non substitué, ou en totalité ou en partie substitué par un ou deux radicaux fluoro, ou
un groupe cycloalkyle en C₃-C₇, le radical cycloalkyle étant non substitué, ou substitué en totalité ou en partie par un ou des radicaux fluoro,
R⁴ et R⁵ représentent chacun indépendamment de l'autre
un atome d'hydrogène,
un groupe alkyle en C₁-C₆, le radical alkyle étant non substitué, ou étant substitué par un ou plusieurs substituants choisis dans le groupe consistant en les radicaux fluoro, chloro, alcoxy en C₁-C₄ ou cycloalkyle en C₃-C₇,
un groupe alcoxy en C₁-C₆, le radical alcoxy ayant une chaîne droite ou ramifiée, et étant non substitué, ou substitué par un ou plusieurs substituants choisis dans le groupe consistant en les radicaux fluoro, chloro, alcoxy en C₁-C₄ ou cycloalkyle en C₃-C₇, et
R⁸ représente
un groupe alkyle en C₁-C₆, le radical alkyle étant non substitué ou en totalité ou en partie substitué par un ou des groupes fluoro,
un groupe cycloalkyle en C₁-C₆, alcényle en C₁-C₆ ou alcoxyalkyle en C₁-C₆, chacun des radicaux mentionnés étant non substitué, ou en totalité ou en partie substitué par un ou des radicaux fluoro,
dans lequel
on fait réagir une 1,3-dihydro-2H-indol-2-one de formule (6-1) 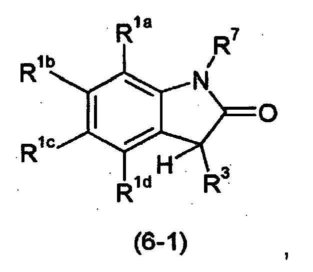
dans laquelle
R^{1a} à R^{1d} sont tels que définis pour la formule (4-1),
R³ représente un atome d'hydrogène, et
R⁷ représente un atome d'hydrogène,
dans une première étape, par
- arylation d'un oxindole à substitution triazinyle de formule (5-1) 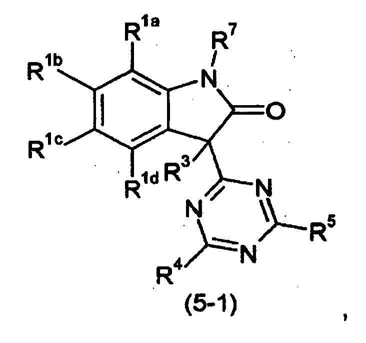 dans laquelle
R^{1a} à R^{1d}, et R⁴ et R⁵ sont tels que définis pour la formule (4-1), et R³ et R⁷ sont tels que définis pour la formule (6-1),
et on fait réagir les produits d'arylation de formule (5-1) dans une deuxième étape, par
- sulfonation pour conduire à des 3-triazinyloxindoles à substitution N-sulfonyle de formule (2-1) 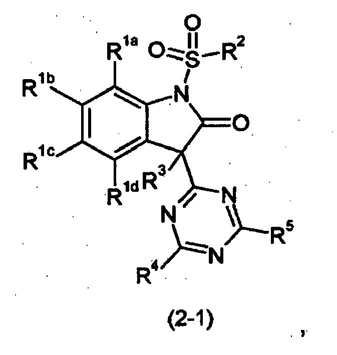 dans laquelle
R^{1a} à R^{1d}, R² à R⁴ et R⁵ sont tels que définis dans la formule (4-1), et R³ est tel que défini pour la formule (6-1),
et on fait réagir les produits de sulfonylation de formule (2-1) dans une troisième étape, par
- décyclisation oxydative conduisant à un 2-(triazinylcarbonyl)sulfonanilide de formule (1-1) 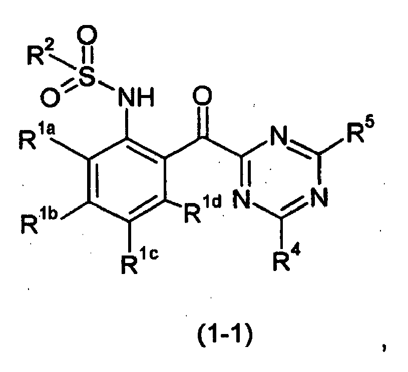 dans laquelle
R^{1a} à R^{1d}, R², ainsi que R⁴ et R⁵, sont tels que définis pour la formule (4-1),
et on fait réagir les produits d'oxydation de formule (1-1) dans une quatrième étape par
- alkylation conduisant à un N-alkyl-N-[2-(1,3,5-triazin-2-ylcarbonyl)phényl]alcanesulfonamide de formule (4-1) 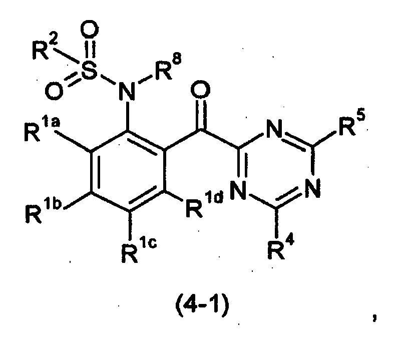
dans laquelle
R^{1a} à R^{1d}, R², R⁴, R⁵ et R⁸ sont tels que définis pour la formule (4-1),
auquel cas on utilise en tant que réactif d'alkylation
- X-R⁸, où X est le chlore, le brome ou l'iode, et R⁸ est tel que défini pour la formule (4-1) ci-dessus, ou
- (R⁸)₂SO₄, où R⁸ est tel que défini ci-dessus pour la formule (4-1).

18. Procédé de production de N-alkyl-N-[2-(1,3,5-riazin-2-ylcarbonyl)phényl]alcanesulfonamides de formule (4-1) selon la revendication 17, **caractérisé en ce qu'**on fait réagir les composés de formule (6-1) utilisés comme matière première dans une étape préalable, en partant d'une 3-(alkylsulfanyl)-1,3-dihydro-2H-indol-2-one de formule (7-1) 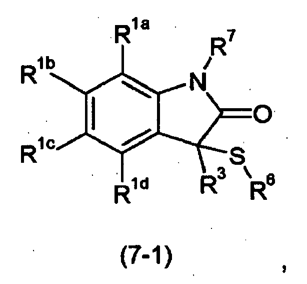 dans laquelle
R^{1a} à R^{1d} sont tels que définis pour la formule (4-1),
R³ représente un atome d'hydrogène,
R⁷ représente un atome d'hydrogène, et
R⁶ est un groupe alkyle en C₁-C₁₄, cycloalkyle en C₃-C₇, benzyle non substitué ou substitué, ou un groupe CH₂-C(O)O-(alkyle en C₁-C₆),
par
- réduction conduisant à une 1,3-dihydro-2H-indol-2-one (6-1) 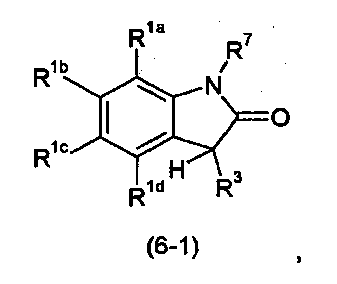 dans laquelle
R^{1a} à R^{1d}, R³ et R⁷ sont tels que définis pour la formule (7-1).
